(19) Europäisches Patentamt
European Patent Office
Office européen des brevets

(11) EP 4 198 026 A1

(12) # EUROPEAN PATENT APPLICATION

(43) Date of publication:
**21.06.2023 Bulletin 2023/25**

(21) Application number: **21214412.5**

(22) Date of filing: **14.12.2021**

(51) International Patent Classification (IPC):
***C07D 239/26*** (2006.01) ***C07D 239/74*** (2006.01)
***C07D 241/12*** (2006.01) ***C07D 251/24*** (2006.01)
***H01L 51/00*** (2006.01)

(52) Cooperative Patent Classification (CPC):
**C07D 251/24; C07D 239/26; C07D 239/74; C07D 241/12; H10K 85/615; H10K 85/654;**
H10K 50/18

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **PAVICIC, Domagoj**
**01099 Dresden (DE)**
- **GALÁN GARCÍA, Elena**
**01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(54) **COMPOUNDS FOR USE IN SEMICONDUCTIONG MATERIALS SUITABLE FOR ELECTRONIC DEVICES**

(57) Compound having the formula (I): E-$A^1$-$A^2$-$A^3$ (I), and to a semiconducting material and to an electronic device comprising the same.

EP 4 198 026 A1

## Description

TECHNICAL FIELD

The present invention relates to an electronic device, especially an organic electronic device such as an organic light-emitting diode, and to a compound, which may be used in the electronic device. The present invention relates further to a semiconducting material comprising the compound.

Organic light-emitting diodes (OLEDs), which are self-emitting devices, have a wide viewing angle, excellent contrast, quick response, high brightness, excellent driving voltage characteristics, and color reproduction. A typical OLED includes an anode, a hole transport layer (HTL), an emission layer (EML), an electron transport layer (ETL), and a cathode, which are sequentially stacked on a substrate. To facilitate electron transport from the cathode into the ETL, the OLED may further comprise an electron injection layer (EIL) arranged between the cathode and the ETL. In this regard, the HTL, the EML, the ETL, and the EIL are thin films formed from organic and / or organometallic compounds.

When a voltage is applied to the anode and the cathode, holes injected from the anode electrode move to the EML, via the HTL, and electrons injected from the cathode electrode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency.

A variety of organic electronic diodes comprising different electron transport materials are well known in the art. For example, WO 2019/238858 A1 discloses tetra-substituted pyrazine compounds as electron transport materials, particularly for hole blocking layers.

However, there is still a need to improve the performance of such devices, in particular to improve the performance of organic electroluminescent devices, particularly with respect to lifetime improvement without impairment of operational voltage and/or device efficiency.

It is, therefore, the object of the present invention to provide an electronic device, such as an organic light emitting diode, overcoming drawbacks of the prior art, in particular an OLED with improved performance, in particular with improved efficiency, more particularly with respect to lifetime improvement without impairment of operational voltage and/or device efficiency.

DISCLOSURE

The object is achieved by a compound having the formula (I)

$$E\text{-}A^1\text{-}A^2\text{-}A^3 \qquad (I),$$

wherein in formula (I)

- E is selected from the group consisting of a substituted or unsubstituted $C_4$-$C_{70}$ heteroaryl comprising a diazine group, a substituted or unsubstituted $C_3$-$C_{70}$ heteroaryl comprising a triazine group, a $C_{10}$-$C_{70}$ aryl comprising at least two condensed benzene rings, and a $C_4$-$C_{70}$ heteroaryl comprising at least two condensed aromatic rings;

  - wherein in case that E is substituted or unsubstituted $C_4$-$C_{70}$ heteroaryl comprising a diazine group E is bonded to $A^1$ via a direct bond between $A^1$ and the diazine group;

  - wherein in case that E is substituted or unsubstituted $C_4$-$C_{70}$ heteroaryl comprising a triazine group E is bonded to $A^1$ via a direct bond between $A^1$ and the triazine group;

  - wherein in case that E is $C_{10}$-$C_{70}$ aryl comprising at least two condensed benzene rings E is bonded to $A^1$ via a direct bond between $A^1$ and one of the condensed benzene rings; and

  - wherein in case that E is $C_4$-$C_{70}$ heteroaryl comprising at least two condensed aromatic rings E is bonded to $A^1$ via a direct bond between $A^1$ and one of the condensed aromatic rings;

- $A^1$ is selected from the group consisting of a substituted or unsubstituted $C_{10}$-$C_{70}$ arylene comprising 2 or 3 condensed rings and a substituted or unsubstituted $C_2$-$C_{70}$ a heteroarylene comprising 2 or 3 condensed rings; wherein $A^1$ is bonded to E via a direct bond between one of the condensed rings and E; and wherein $A^1$ is bonded to $A^2$ via a direct bond between one of the condensed rings and $A^2$;

- $A^2$ is selected from the group consisting of a substituted or unsubstituted carbocyclic aromatic ring, a substituted or unsubstituted heterocyclic aromatic ring, a substituted or unsubstituted fused bicyclic aromatic system and a substituted or unsubstituted fused bicyclic heteroaromatic system; wherein $A^2$ is bonded to $A^3$ via a direct bond between one of the aromatic or heteroaromatic rings and $A^3$ and

- $A^3$ is selected from the group consisting of a monocyclic aryl and a monocyclic heteroaryl, wherein $A^3$ is substituted with at least three groups independently selected from the group consisting of substituted or unsubstituted $C_6$-$C_{18}$ aryl and $C_2$-$C_{18}$ heteroaryl.

The object is further achieved by a semiconducting material comprising the compound of formula (I) according to the present invention.

The object is further achieved by an electronic device device comprising at least one layer comprising the compound of formula (I) according to the present invention.

Finally, the object is achieved by a display device comprising a light emitting diode, wherein the light emitting diode comprises the compound of formula (I) according to the present invention.

Technical effect

It was surprisingly found by the inventors that the compounds of formula (I) according to the present invention allow improving the performance of organic electroluminescent devices comprising the same, particularly with respect to lifetime improvement without impairment of operational voltage and/or device efficiency.

Compound of formula (I) according to the present invention

The compound of formula (I) according to the present invention is represented by the following chemical formula (I).

$$E\text{-}A^1\text{-}A^2\text{-}A^3 \qquad (I).$$

In this compound, E is selected from the group consisting of a substituted or unsubstituted $C_4$-$C_{70}$ heteroaryl comprising a diazine group, a substituted or unsubstituted $C_3$-$C_{70}$ heteroaryl comprising a triazine group, a $C_{10}$-$C_{70}$ aryl comprising at least two condensed benzene rings, and a $C_4$-$C_{70}$ heteroaryl comprising at least two condensed aromatic rings. E can be considered as a charge transport structural moiety.

If it is described herein that a specific group, such as the group E or another group described herein, such as $A^1$, $A^2$, $A^3$ etc., is substituted, the one or more substituents may be independently selected from the group consisting of $C_6$ to $C_{12}$ aryl, $C_3$ to $C_{11}$ heteroaryl, $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN and $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, if not explicitly mentioned else, that is, if no limited selection of possible substituents is mentioned in a specific context.

E and $A^1$ are connected with each other via a direct bond. In this regard, a direct bond is a single bond.

In case that E is substituted or unsubstituted $C_4$-$C_{70}$ heteroaryl comprising a diazine group E is bonded to $A^1$ via a direct bond between $A^1$ and the diazine group. For example, in case that E is triphenyldiazine and $A^1$ is naphthylene, the binding situation may be as follows:

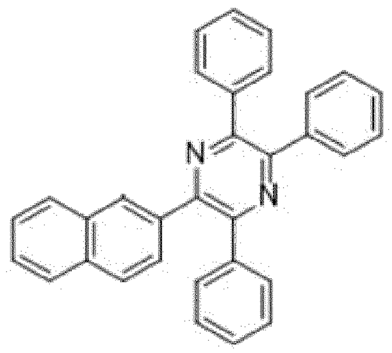

In case that E is substituted or unsubstituted $C_4$-$C_{70}$ heteroaryl comprising a triazine group E is bonded to $A^1$ via a direct bond between $A^1$ and the triazine group. For example, in case that E is biphenyltriazine and $A^1$ is naphthylene, the binding situation may be as follows:

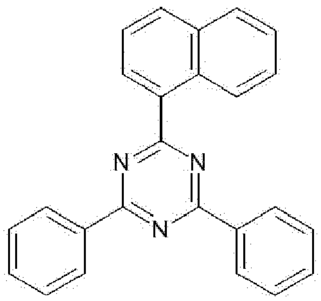

In case that E is $C_{10}$-$C_{70}$ aryl comprising at least two condensed benzene rings E is bonded to $A^1$ via a direct bond between $A^1$ and one of the condensed benzene rings. Likewise, in case that E is $C_4$-$C_{70}$ heteroaryl comprising at least two condensed aromatic rings E is bonded to $A^1$ via a direct bond between $A^1$ and one of the condensed aromatic rings. For example, in case that E is phenyl-quinazoline and $A^1$ is naphthylene, the binding situation may be as follows:

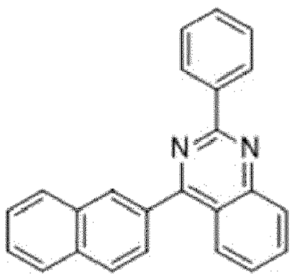

Likewise, $A^1$ and $A^2$ are connected with each other via a bond and $A^2$ and $A^3$ are connected with each other via a bond.

$A^1$ is selected from the group consisting of a substituted or unsubstituted $C_{10}$-$C_{70}$ arylene comprising 2 or 3 condensed rings and a substituted or unsubstituted $C_2$-$C_{70}$ a heteroarylene comprising 2 or 3 condensed rings; wherein $A^1$ is bonded to E via a direct bond between one of the condensed rings and E; and wherein $A^1$ is bonded to $A^2$ via a direct bond between one of the condensed rings and $A^2$.

$A^2$ is selected from the group consisting of a substituted or unsubstituted carbocyclic aromatic ring, a substituted or unsubstituted heterocyclic aromatic ring, a substituted or unsubstituted condensed bicyclic aromatic system and a substituted or unsubstituted condensed bicyclic heteroaromatic system; wherein $A^2$ is bonded to $A^3$ via a direct bond between one of the aromatic or heteroaromatic rings and $A^3$.

In condensed (also called "fused") bicyclic compounds (or bicyclic moieties), two rings share two adjacent atoms.

$A^3$ is selected from the group consisting of a monocyclic aryl and a monocyclic heteroaryl, wherein $A^3$ is substituted with at least three groups independently selected from the group consisting of substituted or unsubstituted $C_6$-$C_{18}$ aryl, especially phenyl, and $C_2$-$C_{18}$ heteroaryl, especially pyridinyl. In this regard, the number of substituents (at least three) does not include the group $A^2$. In other words, $A^3$ has at least three substituents besides $A^2$ ($A^2$ in this context including the moieties $A^1$ and E attached thereto).

E may be selected from the group consisting of a substituted or unsubstituted $C_4$-$C_{60}$ heteroaryl comprising a diazine group, a substituted or unsubstituted $C_3$-$C_{60}$ heteroaryl comprising a triazine group, a $C_{10}$-$C_{60}$ aryl comprising at least two condensed benzene rings, and a $C_4$-$C_{60}$ heteroaryl comprising at least two condensed aromatic rings. E may be selected from the group consisting of a substituted or unsubstituted $C_4$-$C_{50}$ heteroaryl comprising a diazine group, a substituted or unsubstituted $C_3$-$C_{50}$ heteroaryl comprising a triazine group, a $C_{10}$-$C_{50}$ aryl comprising at least two condensed benzene rings, and a $C_4$-$C_{50}$ heteroaryl comprising at least two condensed aromatic rings. E may be selected from the group consisting of a substituted or unsubstituted $C_4$-$C_{52}$ heteroaryl comprising a diazine group, a substituted or unsubstituted $C_3$-$C_{51}$ heteroaryl comprising a triazine group, a $C_{10}$-$C_{58}$ aryl comprising at least two condensed benzene rings, and a $C_4$-$C_{57}$ heteroaryl comprising at least two condensed aromatic rings. E may be selected from the group consisting of a substituted or unsubstituted $C_4$-$C_{46}$ heteroaryl comprising a diazine group, a substituted or unsubstituted $C_3$-$C_{45}$ heteroaryl comprising a triazine group, a $C_{10}$-$C_{52}$ aryl comprising at least two condensed benzene rings, and a $C_4$-$C_{51}$ heteroaryl comprising at least two condensed aromatic rings. E may be selected from the group consisting of a substituted or unsubstituted $C_4$-$C_{40}$ heteroaryl comprising a diazine group, a substituted or unsubstituted $C_3$-$C_{39}$ heteroaryl comprising a triazine group, a $C_{10}$-$C_{46}$ aryl comprising at least two condensed benzene rings, and a $C_4$-$C_{45}$ heteroaryl comprising at least two condensed aromatic rings. E may be selected from the group consisting of a substituted or unsubstituted $C_4$-$C_{34}$ heteroaryl comprising a diazine group, a substituted or unsubstituted $C_3$-$C_{33}$ heteroaryl comprising a triazine group, a $C_{10}$-$C_{40}$ aryl comprising at least two condensed benzene rings, and a $C_4$-$C_{39}$ heteroaryl comprising at least two condensed aromatic rings. E may be selected from the group consisting of a substituted or unsubstituted $C_4$-$C_{28}$ heteroaryl comprising a diazine group, a substituted or unsubstituted $C_3$-$C_{27}$ heteroaryl comprising a triazine group, a $C_{10}$-$C_{34}$ aryl comprising at least two condensed benzene rings, and a $C_4$-$C_{33}$ heteroaryl comprising at least two condensed aromatic rings. E may be selected from the group consisting of a substituted or unsubstituted $C_4$-$C_{22}$ heteroaryl comprising a diazine group, a substituted or unsubstituted $C_3$-$C_{21}$ heteroaryl comprising a triazine group, a $C_{10}$-$C_{28}$ aryl comprising at least two condensed benzene rings, and a $C_4$-$C_{27}$ heteroaryl comprising at least two condensed aromatic rings. E may be selected from the group consisting of a substituted or unsubstituted $C_4$-$C_{16}$ heteroaryl comprising a diazine group, a substituted or unsubstituted $C_3$-$C_{15}$ heteroaryl comprising a triazine group, a $C_{10}$-$C_{22}$ aryl comprising at least two condensed benzene rings, and a $C_4$-$C_{21}$ heteroaryl comprising at least two condensed aromatic rings.

E may be selected from the group consisting of pyrimidine, pyrazine, pyridazine, triazine, naphthalene and aza-analogs thereof, especially quinazoline, anthracene and aza-analogs thereof, phenanthrene and aza-analogs thereof, specially benzoquinazoline, fluorene and aza-analogs thereof, benzofluorene and aza-analogs thereof, dibenzofluorene and aza-analogs thereof, naphtofluorene and aza-analogs thereof, benzo-naphtofluorene and aza-analogs thereof, dinaphtofluorene and aza-analogs thereof, benzimidazole and aza analogs thereof, benzoxazole and aza analogs thereof, benzothiazole and aza analogs thereof, pyrido-imidazole and aza analogs thereof, benzofurane and aza-analogs thereof, benzothiophene and aza-analogs thereof, dibenzofurane and aza-analogs thereof, dibenzothiophene and aza-analogs thereof, naphtofurane and aza-analogs thereof, naphtothiophene and aza-analogs thereof, naphtobenzofurane and aza-analogs thereof, naphtobenzothiophene and aza-analogs thereof, dinaphtofurane and aza-analogs thereof, dinaphtothiophene and aza-analogs thereof, aryl consisting of 4, 5, 6 or 7 condensed rings and aza-analogs thereof, and heteroaryl consisting of 3, 4, 5, 6 or 7 condensed rings and comprising 1, 2 or 3 ring atoms independently selected from O and S, and aza-analogs of the heteroaryl consisting of 3, 4, 5, 6 or 7 condensed rings and comprising 1, 2 or 3 ring atoms independently selected from O and S, wherein the groups may be substituted or unsubstituted respectively, especially with one or more phenyl and/or pyridyl groups, wherein the phenyl and/or pyridyl groups may be substituted or unsubstituted.

The term "aza-analog" as used herein refers to a respective group in which one or more of the carbon atoms of the group are replaced by nitrogen atoms. For example, aza-analogs of naphthalene

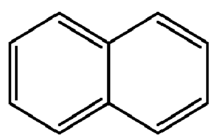

include quinoline and quinazoline

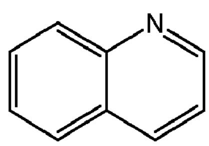

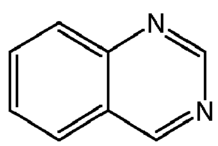

.

E may be selected from the group consisting of pyrimidine, pyrazine, cinnoline, quioline, phtalazine, quinoxaline, quinazoline, benzoquinazoline, pyrido-imidazole, benzimidazole, pyrido-benzimidazole and 1,3,5-triazine, wherein the groups may be substituted or unsubstituted, respectively.

E may be selected from the group consisting of pyrimidine, pyrazine, quinazoline, benzoquinazoline, and 1,3,5-triazine, wherein the groups may be substituted or unsubstituted, respectively, especially may be substituted with one or more unsubstituted phenyl.

E (including the substituents) may be selected from the group consisting of

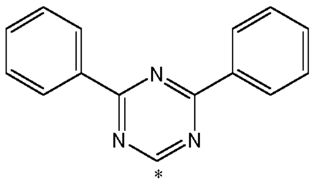 ; 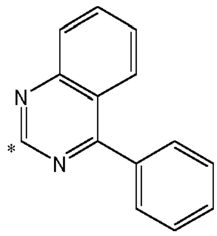 ; 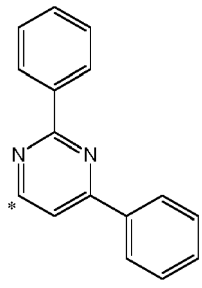 ; 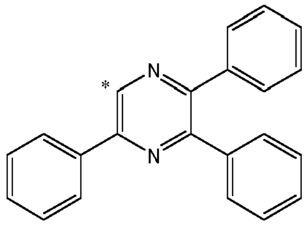 ;

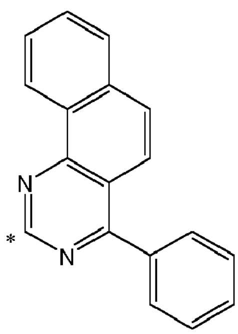 ; and 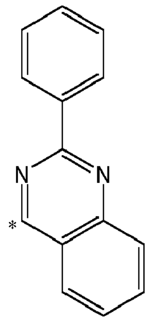 ;

wherein the asterisk symbol "*" represents the binding position of the group to $A^1$.

$A^1$ may be selected from the group consisting of a substituted or unsubstituted $C_{10}$-$C_{58}$ arylene comprising 2 or 3 condensed rings and a substituted or unsubstituted $C_2$-$C_{57}$ a heteroarylene comprising 2 or 3 condensed rings. $A^1$ may be selected from the group consisting of a substituted or unsubstituted $C_{10}$-$C_{52}$ arylene comprising 2 or 3 condensed rings and a substituted or unsubstituted $C_2$-$C_{51}$ a heteroarylene comprising 2 or 3 condensed rings. $A^1$ may be selected from the group consisting of a substituted or unsubstituted $C_{10}$-$C_{46}$ arylene comprising 2 or 3 condensed rings and a substituted or unsubstituted $C_2$-$C_{45}$ a heteroarylene comprising 2 or 3 condensed rings. $A^1$ may be selected from the group consisting of a substituted or unsubstituted $C_{10}$-$C_{40}$ arylene comprising 2 or 3 condensed rings and a substituted or unsubstituted $C_2$-$C_{39}$ a heteroarylene comprising 2 or 3 condensed rings. $A^1$ may be selected from the group consisting of a substituted or unsubstituted $C_{10}$-$C_{34}$ arylene comprising 2 or 3 condensed rings and a substituted or unsubstituted $C_2$-$C_{33}$ a heteroarylene comprising 2 or 3 condensed rings. $A^1$ may be selected from the group consisting of a substituted or unsubstituted $C_{10}$-$C_{28}$ arylene comprising 2 or 3 condensed rings and a substituted or unsubstituted $C_2$-$C_{27}$ a heteroarylene comprising 2 or 3 condensed rings. $A^1$ may be selected from the group consisting of a substituted or unsubstituted $C_{10}$-$C_{22}$ arylene comprising 2 or 3 condensed rings and a substituted or unsubstituted $C_2$-$C_{21}$ a heteroarylene comprising 2 or 3 condensed rings. $A^1$ may be selected from the group consisting of a substituted or unsubstituted $C_{10}$-$C_{16}$ arylene comprising 2 or 3 condensed rings and a substituted or unsubstituted $C_2$-$C_{15}$ a heteroarylene comprising 2 or 3 condensed rings.

$A^1$ may be selected from the group consisting of a substituted or unsubstituted $C_{10}$-$C_{58}$ arylene comprising 2 or 3 condensed rings. $A^1$ may be selected from the group consisting of a substituted or unsubstituted $C_{10}$-$C_{52}$ arylene comprising 2 or 3 condensed rings. $A^1$ may be selected from the group consisting of a substituted or unsubstituted $C_{10}$-$C_{46}$ arylene comprising 2 or 3 condensed rings. $A^1$ may be selected from the group consisting of a substituted or unsubstituted $C_{10}$-$C_{40}$ arylene comprising 2 or 3 condensed rings. $A^1$ may be selected from the group consisting of a substituted or unsubstituted $C_{10}$-$C_{34}$ arylene comprising 2 or 3 condensed rings. $A^1$ may be selected from the group consisting of a substituted or unsubstituted $C_{10}$-$C_{28}$ arylene comprising 2 or 3 condensed rings. $A^1$ may be selected from the group consisting of a substituted or unsubstituted $C_{10}$-$C_{22}$ arylene comprising 2 or 3 condensed rings. $A^1$ may be selected from the group consisting of a substituted or unsubstituted $C_{10}$-$C_{16}$ arylene comprising 2 or 3 condensed rings.

$A^1$ may selected from the group consisting of naphtalene-diyl and aza-analogs thereof, anthracene-diyl and

aza-analogs thereof, phenanthrene-diyl and aza-analogs thereof, fluorene-diyl and aza-analogs thereof, benzothiophene-diyl and aza-analogs thereof, benzofurane-diyl and aza-analogs thereof, naphtofurane-diyl and aza-analogs thereof, naphtothiophene-diyl and aza-analogs thereof, dibenzofurane-diyl aza-analogs thereof, and dibenzothiophene-diyl and aza-analogs thereof.

$A^1$ may selected from the group consisting of naphtalene-diyl and aza-analogs thereof, and phenanthrene-diyl and aza-analogs thereof.

$A^1$ may be selected from the group consisting of naphtalene-1,2-diyl, naphtalene-1,3-diyl, naphtalene-1,4-diyl, naphtalene-1,5-diyl, naphtalene-1,6-diyl, naphtalene-1,7-diyl, naphtalene-1,8-diyl, naphtalene-2,3-diyl, and naphtalene-2,6-diyl, naphtalene-2,7-diyl, and phenanthrene-9,10-diyl.

$A^2$ may be selected from the group consisting of phenylene, thiophenylene, furanylene, pyrazolylene, imidazolylene, triazolylene, pyridinylene, pyrimidinylene, pyridazinylene, pyrazinelyen and triazinylene.

$A^2$ may be selected from the group consisting of o-phenylene, m-phenylene, p-phenylene, and pyridine-3,5-ylene.

$A^3$ (not including the at least three groups by which $Ar^3$ is substituted) may be selected from the group consisting of phenyl, thiophenyl, furanyl, pyridinyl, pyrimidinyl, pyridazinyl, and pyrazinyl.

$A^3$ (not including the at least three groups by which $Ar^3$ is substituted) may be selected from the group consisting of phenyl, pyridinyl, pyrimidinyl and pyrazinyl.

$A^3$ (not including the at least three groups by which $Ar^3$ is substituted) may be selected from the group consisting of phenyl, pyrimidinyl and pyrazinyl.

The at least three groups by which $A^3$ is substituted may be independently selected from the group consisting of unsubstituted phenyl, phenyl substituted with one or more hydrocarbyl groups, wherein the total number of carbon atoms in the phenyl substituted with one or more hydrocarbyl groups is from 7 to 18, pyridyl, pyridyl substituted with one or more hydrocarbyl groups, wherein the total number of carbon atoms in the phenyl substituted with one or more hydrocarbyl groups is from 7 to 18, and CN-substituted phenyl.

The at least three groups by which $A^3$ is substituted may be independently selected from the group consisting of unsubstituted phenyl, CN-substituted phenyl, especially meta-CN-substituted phenyl, pyridyl, and picoline (methylpyridyl, for example 2-methylpyridyl, 3-methylpyridyl or 4-methylpyridyl).

The at least three groups by which $A^3$ is substituted may be independently selected from the group consisting of

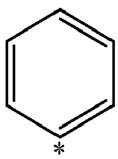 ; 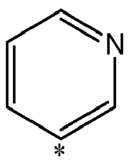 ; 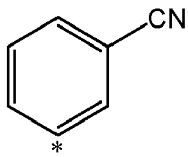 ; and 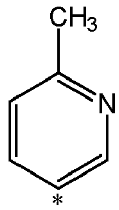 .

wherein the asterisk symbol "*" represents the binding position of the group to $A^3$.

$A^3$ (including the at least three groups by which $Ar^3$ is substituted) may be selected from the group consisting of

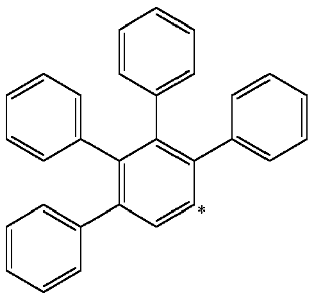 ; 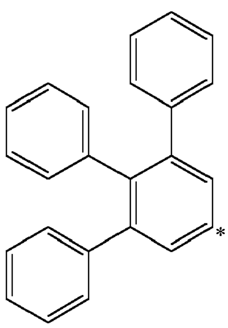 ; 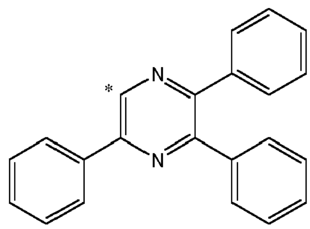 ;

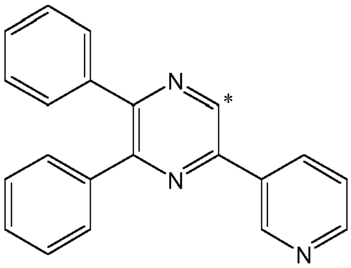 ; 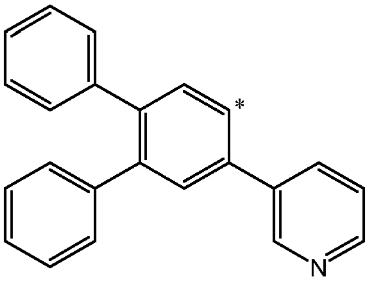 ; 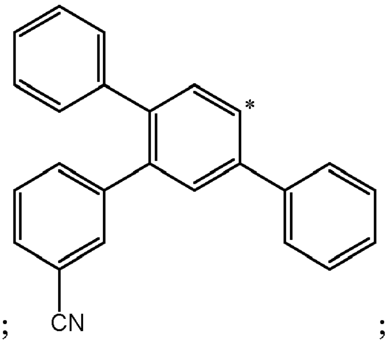 ;

and

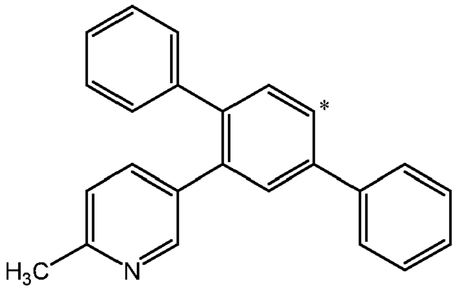 ;

wherein the asterisk symbol "*" represents the binding position of the group to $A^2$.

The compound of formula (I) may be selected from the following compounds E1 to E20

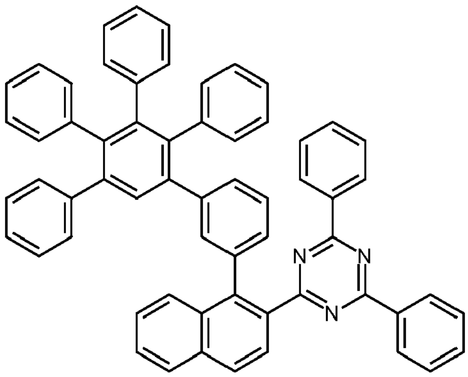

E1

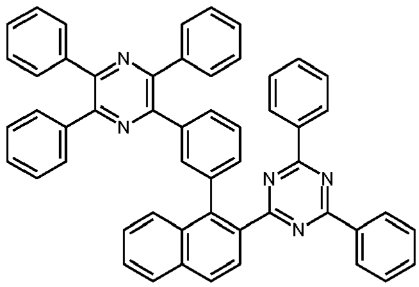

E2

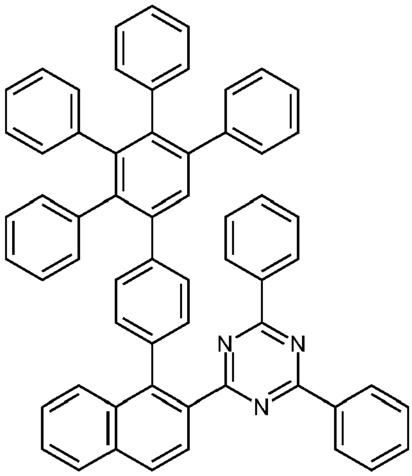

E3

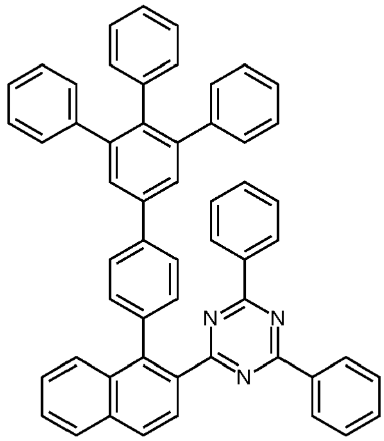

E4

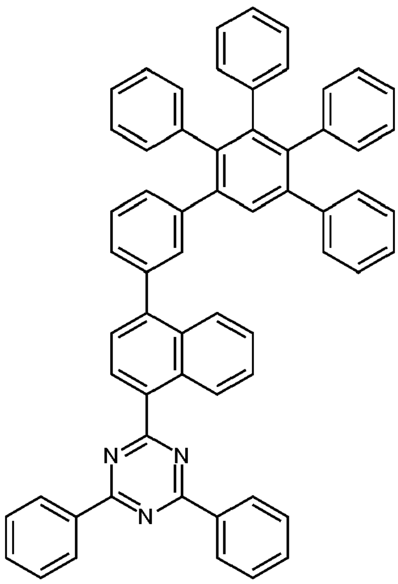

E5

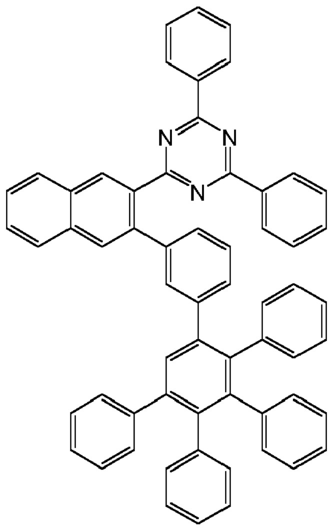

E6

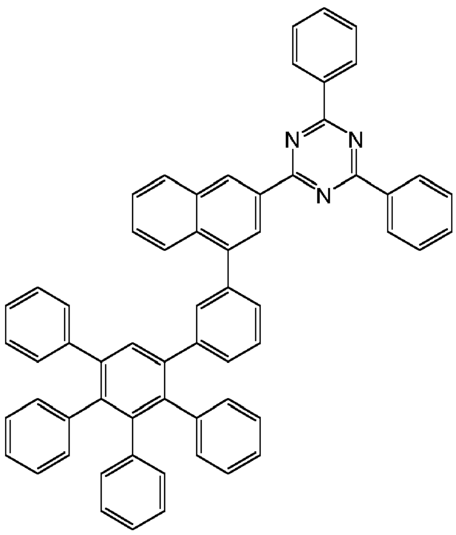

E7

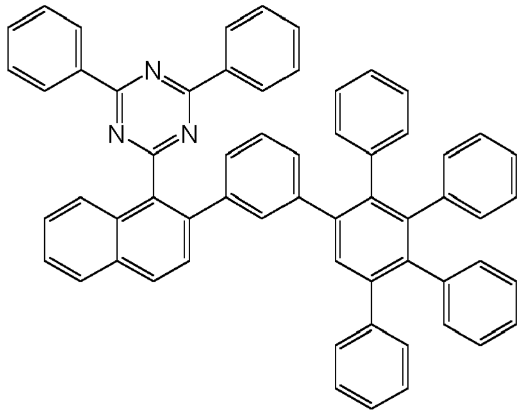

E8

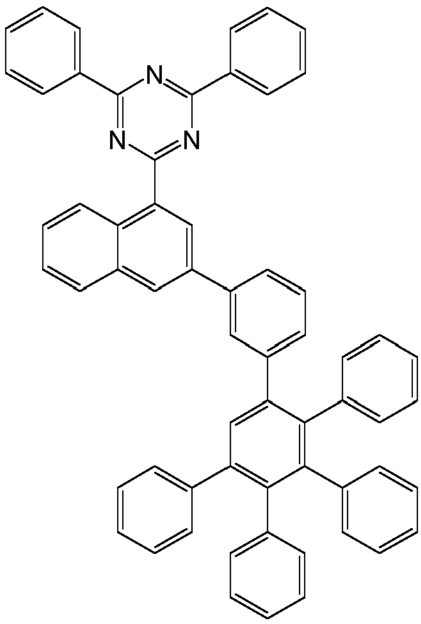

E9

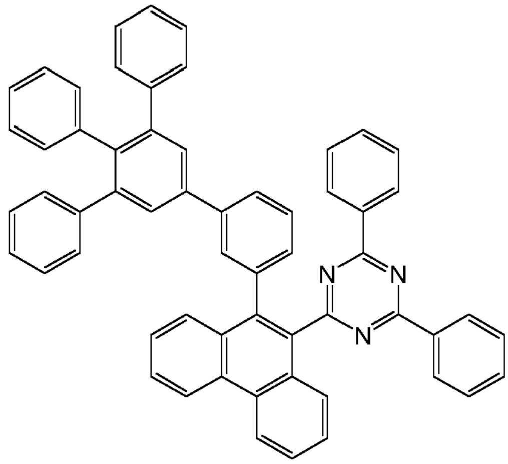

E10

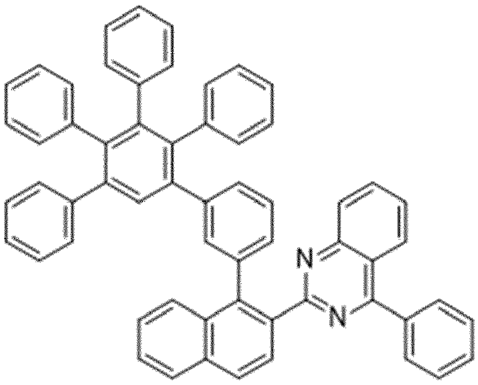

E11

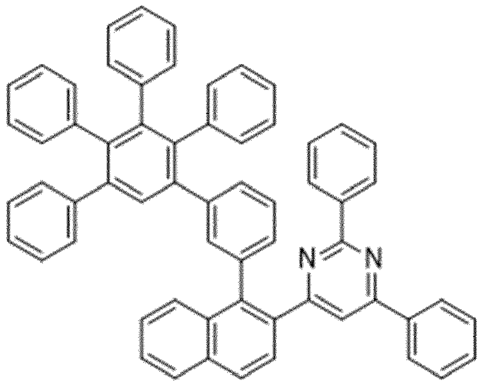

E12

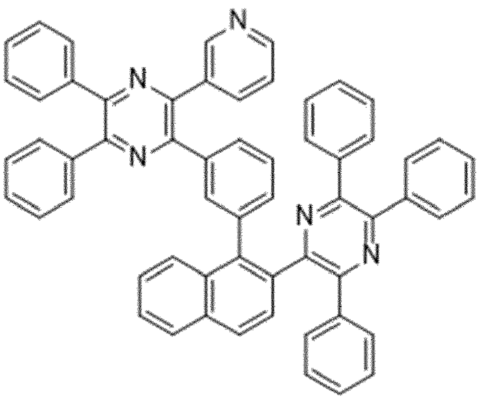

E13

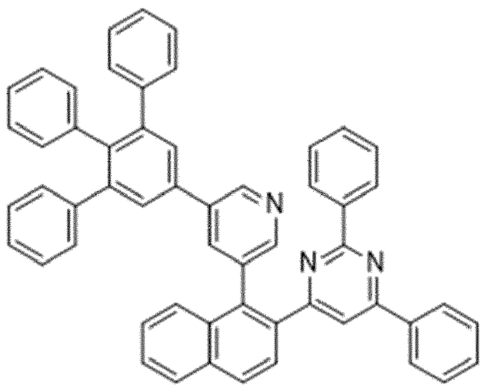

E14

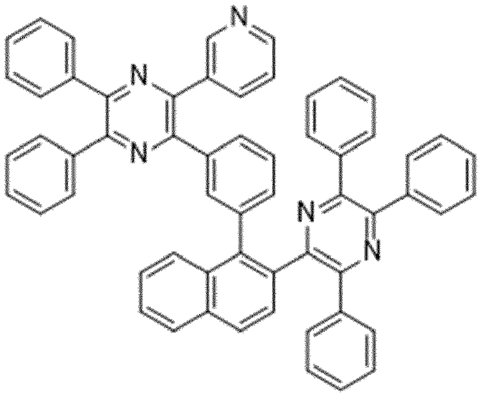

E15

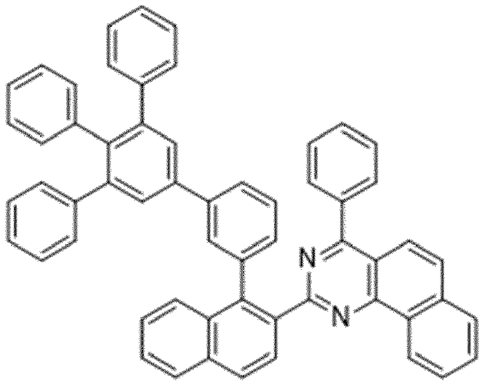

E16

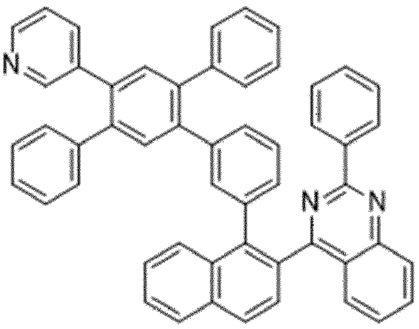

E17

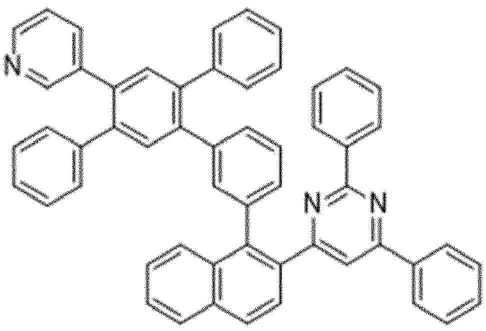

E18

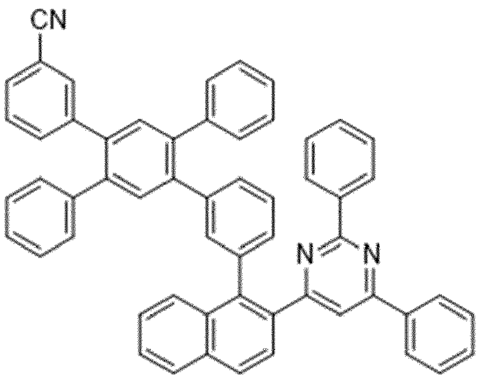

E19

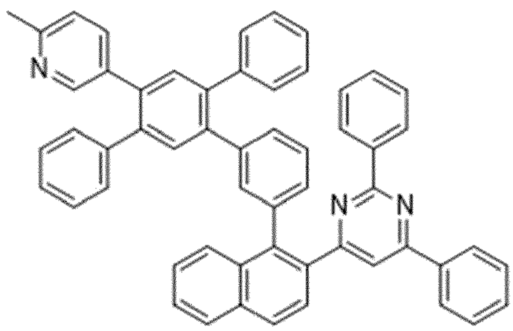

E20.

Specific embodiments of the compound of formula (I)

In the following specific embodiments of the compound of formula (I) are described wherein these embodiments may be combined with one or more of the above features.

In one embodiment, the invention relates to a compound having the formula (I)

$$E\text{-}A^1\text{-}A^2\text{-}A^3 \qquad (I),$$

wherein in formula (I)

- E is selected from the group consisting of a substituted or unsubstituted $C_4$-$C_{16}$ heteroaryl comprising a diazine group, a substituted or unsubstituted $C_3$-$C_{15}$ heteroaryl comprising a triazine group, a $C_{10}$-$C_{22}$ aryl comprising at least two condensed benzene rings, and a $C_4$-$C_{21}$ heteroaryl comprising at least two condensed aromatic rings;
- $A^1$ is selected from the group consisting of a substituted or unsubstituted $C_{10}$-$C_{16}$ arylene comprising 2 or 3 condensed rings and a substituted or unsubstituted $C_2$-$C_{15}$ a heteroarylene comprising 2 or 3 condensed rings;

- $A^2$ is selected from the group consisting of phenylene, thiophenylene, furanylene, pyrazolylene, imidazolylene, triazolylene, pyridinylene, pyrimidinylene, pyridazinylene, pyrazinelyen and triazinylene; and

- $A^3$ is selected from the group consisting of phenyl, thiophenyl, furanyl, pyridinyl, pyrimidinyl, pyridazinyl, and pyrazinyl, wherein $Ar^3$ is substituted with at least three groups, wherein the at least three groups by which $A^3$ is substituted are independently selected from the group consisting of unsubstituted phenyl, phenyl substituted with one or more hydrocarbyl groups, wherein the total number of carbon atoms in the phenyl substituted with one or more hydrocarbyl groups is from 7 to 18, pyridyl, pyridyl substituted with one or more hydrocarbyl groups, wherein the total number of carbon atoms in the phenyl substituted with one or more hydrocarbyl groups is from 7 to 18, and CN-substituted phenyl;

wherein the groups E, $A^1$, $A^2$ and $A^3$ are connected with each other via direct bonds (single bonds) as set forth above.

In one embodiment, the invention relates to a compound having the formula (I)

$$E\text{-}A^1\text{-}A^2\text{-}A^3 \qquad (I),$$

wherein in formula (I)

- E is selected from the group consisting of pyrimidine, pyrazine, cinnoline, quinoline, phtalazine, quinoxaline, quinazoline, benzoquinazoline, pyrido-imidazole, benzimidazole, pyrido-benzimidazole and 1,3,5-triazine, wherein the groups may be substituted or unsubstituted, respectively;

- $A^1$ is selected from the group consisting of naphtalene-diyl and aza-analogs thereof, anthracene-diyl and aza-analogs thereof, phenanthrene-diyl and aza-analogs thereof, fluorene-diyl and aza-analogs thereof, benzothiophene-diyl and aza-analogs thereof, benzofurane-diyl and aza-analogs thereof, naphtofurane-diyl and aza-analogs thereof, naphtothiophene-diyl and aza-analogs thereof, dibenzofurane-diyl aza-analogs thereof, and dibenzothiophene-diyl and aza-analogs thereof;

- $A^2$ is selected from the group consisting of phenylene, thiophenylene, furanylene, pyrazolylene, imidazolylene, triazolylene, pyridinylene, pyrimidinylene, pyridazinylene, pyrazinelyen and triazinylene; and

- $A^3$ is selected from the group consisting of phenyl, pyridinyl, pyrimidinyl and pyrazinyl; wherein $Ar^3$ is substituted with at least three groups, wherein the at least three groups by which $A^3$ is substituted are independently selected from the group consisting of unsubstituted phenyl, CN-substituted phenyl, especially meta-CN-substituted phenyl, pyridyl, and picoline (2-methylpyridyl);

wherein the groups E, $A^1$, $A^2$ and $A^3$ are connected with each other via direct bonds (single bonds) as set forth above.

In one embodiment, the invention relates to a compound having the formula (I)

$$E\text{-}A^1\text{-}A^2\text{-}A^3 \qquad (I),$$

wherein in formula (I)

- E (including the substituents) is selected from the group consisting of

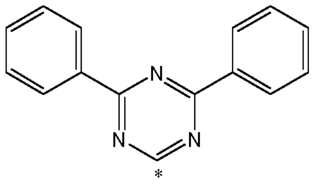; 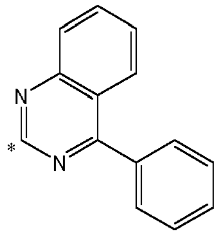; 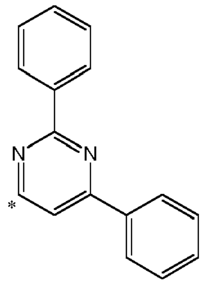; 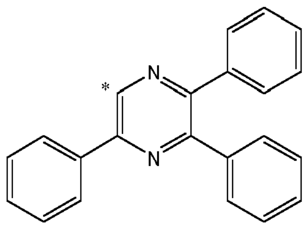;

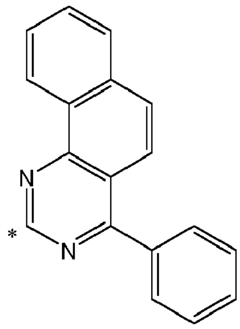; and 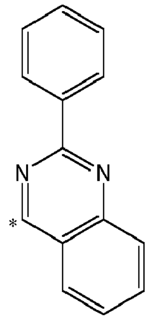;

$A^1$ is selected from the group consisting of naphtalene-1,2-diyl, naphtalene-1,3-diyl, naphtalene-1,4-diyl, naphtalene-1,5-diyl, naphtalene-1,6-diyl, naphtalene-1,7-diyl, naphtalene-1,8-diyl, naphtalene-2,3-diyl, and naphtalene-2,6-diyl, naphtalene-2,7-diyl, and phenanthrene-9,10-diyl;

$A^2$ is selected from the group consisting of o-phenylene, m-phenylene, p-phenylene, and pyridine-3,5-ylene; and

$A^3$ is substituted may be independently selected from the group consisting of

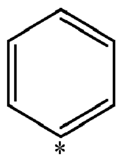; 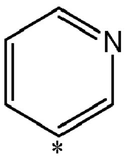; 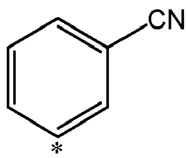; and 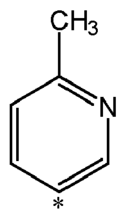;

wherein the asterisk symbol "*" represents the binding position of the group to $A^1$;

wherein the groups E, $A^1$, $A^2$ and $A^3$ are connected with each other via direct bonds (single bonds) as set forth above.

Semiconducting material

The present invention is further related to a semiconducting material comprising the compound of formula (I) according to the present invention as defined herein. Embodiments with respect to the compound per se are also embodiments for the semiconducting material comprising said compound.

The semiconducting material may be an electron transport material, that is, a material allowing, for example, in an OLED, transport of electrons from a cathode to a light emitting layer through the electron transport material.

The semiconducting material may comprise the compound of formula (I) (or two or more of respective compounds) and a further material not having the formula (I).

The semiconducting material may be an electron transport material and the compound of formula (I) may be an electron transport matrix. In this regard, it may be provided that the compound of formula (I) of formula (I) is the predominant material in the semiconducting material, especially may be doped with at least one electrical n-dopant.

In this regard, electrical n-dopants are in particular, but not limited thereto, an elemental metal, alternatively an electropositive metal selected from alkali metals, alkaline earth metals, rare earth metals and transition metals, transition

metals; a metal salt, alternatively an alkali metal salt, alkaline earth metal salt and/or rare earth metal salt, or a metal complex, alternatively an alkali metal complex, alkaline earth metal complex, transition metal complex and/or rare earth metal complex. Examples of n-doping metal salts can be LiF, LiCl, LiBr, LiI, metal borates, metal quinolinolates or mixtures thereof. Further examples of electrical n-dopants are strong chemical reducing agents. This class of "redox" n-dopants may be generically characterized by energy level of the highest occupied molecular orbital (HOMO) comparable with lowest unoccupied molecular orbital energy level of corresponding electron transport matrices, which can, in usual OLED transport materials, have a value about -3.0 eV or less negative.

Electrical n-dopants may be organic compounds as disclosed in EP1837926A1, WO07107306A1 or WO07107356A1.

## Electronic device

The invention further relates to an electronic device comprising at least one layer comprising the compound of formula (I) according to the present invention as defined herein. Embodiments with respect to the compound per se are also embodiments for the semiconducting material comprising said compound.

The electronic device according to the present invention may be an organic electronic device. The organic electronic device according to the invention may be an organic light-emitting device.

The organic electronic device may comprise an electron transport layer, wherein the compound of formula (I) is comprised in the electron transport layer.

The organic electronic device may comprise a cathode, a light emitting layer and an electron transport layer, wherein the electron transport layer is arranged between the cathode and the light emitting layers, and the compound of formula (I) is comprised in the electron transport layer.

The organic electronic device may comprise a cathode, a light emitting layer and an electron transport layer, wherein the electron transport layer is arranged between the cathode and the light emitting layer, and the compound of formula (I) is comprised in the electron transport layer.

The organic electronic device may comprise a cathode, a light emitting layer and an electron transport layer, wherein the electron transport layer is arranged between the cathode and the light emitting layer, the electron transport layer is in direct contact with the light emitting layer and the compound of formula (I) is comprised in the electron transport layer.

According to one aspect of the present invention, there is provided an organic light-emitting diode (OLED) comprising: a substrate; an anode electrode formed on the substrate; a hole injection layer, a hole transport layer, an emission layer, an electron transport layer, the electron injection layer and a cathode electrode, wherein the compound of formula (I) is comprised in the electron transport layer or in the electron injection layer.

According to one aspect of the present invention, there is provided an organic light-emitting diode (OLED) comprising: a substrate; an anode electrode formed on the substrate; a hole injection layer, a hole transport layer, an electron blocking layer, an emission layer, an electron transport layer, the electron injection layer and a cathode electrode, wherein the compound of formula (I) is comprised in the electron transport layer or in the electron injection layer.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer, the electron injection layer and a cathode electrode, wherein the compound of formula (I) is comprised in hole blocking layer, the electron transport layer or in the electron injection layer, especially in the hole blocking layer.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer, an electron injection layer, and a cathode electrode, wherein the compound of formula (I) is comprised in hole blocking layer, the electron transport layer or in the electron injection layer, especially in the hole blocking layer.

## Display device

The present invention further relates to a display comprising a light emitting diode, wherein the light emitting diode comprises the compound of formula (I) according to the present invention as defined herein. Embodiments with respect to the compound per se are also embodiments for the semiconducting material comprising said compound.

## Further layers

In accordance with the invention, the (organic) electronic device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys. The transparent or semitransparent anode may facilitate light emission through the anode.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene)-dimalononitrile, 4,4',4"-((1E,1'E,1"E)-cyclopropane-1,2,3-triylidenetris(cyanomethanylylidene))tris(2,3,5,6-tetrafluorobenzonitrile) or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-1-yl)-N,N'-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010

and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are $Ir(ppy)_3$ (ppy = phenylpyridine), $Ir(ppy)_2(acac)$, $Ir(mpyp)_3$.

Examples of phosphorescent blue emitter dopants are $F_2Irpic$, $(F_2ppy)_2Ir(tmd)$ and $Ir(dfppz)_3$ and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the

EML comprises a phosphorescent dopant, the HBL may have also a triplet exciton blocking function. The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The compound of formula (I) may comprised in the HBL.

*Electron transport layer (ETL)*

The OLED according to the present invention may comprise an electron transport layer (ETL).

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer may comprise suitable ETM materials known in the art. In accordance with the invention, the electron transport layer may comprise, besides the ETM material, at least one additive as defined herein.

Further, the electron transport layer may (or may not) comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another embodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

The compound of formula (I) may comprised in the electron transport layer.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, $Li_2O$, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

The EIL may consist of a number of individual EIL sublayers. In case the EIL consists of a number of individual EIL sublayers, the number of sublayers is preferably 2. The individual EIL sublayers may comprise different materials for forming the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL if present, preferably directly on the EIL, preferably in direct contact with the EIL. In the sense of this invention the cathode and the EIL can be regarded as one functional part enabling the injection of electrons into the electron transport layer. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium

(Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy. The transparent or semitransparent cathode may facilitate light emission through the cathode.

It is to be understood that the cathode electrode is not part of the electron injection layer.

*Charge generation layer*

A charge generation layer (CGL) may be comprised in the inventive OLED. This layer may comprise a p-type CGL and an n-type CGL. An interlayer may be arranged between the p-type layer and the n-type layer.

Typically, the charge generation layer GCL is a pn-junction joining an n-type charge generation layer (electron generating layer, n-type CGL) and a-type charge generation layer (hole generating layer, p-type CGL). The n-side of the pn-junction generates electrons and injects them into the layer which is adjacent in the direction to the anode. Analogously, the p-side of the pn-junction generates holes and injects them into the layer which is adjacent in the direction to the cathode.

Charge generation layers are used in tandem OLEDs comprising, between the cathode and anode, two or more emission layers. In a tandem OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, $FeCl_3$, $FeF_3$, and $SbCl_5$. Also, the host can be one selected from a group consisting of N,N'-di(naphthalen-i-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generation layer may be layer of a neat n-type dopant, for example of an electropositive metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Cs, K, Rb, Mg, Na, Ca, Sr, Eu and Yb. Suitable matrix materials for the n-type charge generation layer layer may be the materials conventionally used as matrix materials for electron injection or electron transport layers. The matrix material can be for example one selected from a group consisting of N-containing heterocyclic compounds like triazine compounds or phenathroline compounds such as compound E or bipyridyl or terpyridyl compounds, hydroxyquinoline derivatives like tris(8-hydroxyquinoline)aluminum, benzazole derivatives, and silole derivatives.

Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an electron transport layer, the electron injection layer and a cathode electrode.

According to one aspect of the present invention, there is provided an organic light-emitting diode (OLED) comprising: a substrate; an anode electrode formed on the substrate; a hole injection layer, a hole transport layer, an electron blocking layer, an emission layer, an electron transport layer, the electron injection layer and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer, the electron injection layer and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer, an electron injection layer, and a cathode electrode.

According to various embodiments of the present invention, there may be provided OLED layers arranged between the above mentioned layers, on the substrate or on the top electrode.

According to one aspect, the OLED can comprise a layer structure of a substrate that is adjacent arranged to

an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to an electron transport layer, which may optionally be formed by two sub-layer, which are a first electron transport layer and a second electron transport layer, the first electron transport layer is adjacent arranged to a second electron transport layer, the second electron transport layer is adjacent arranged to an electron injection layer, the electron injection layer is adjacent arranged to the cathode electrode.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, the method may further include forming on the anode electrode, an emission layer and at least one layer selected from the group consisting of forming a hole injection layer, forming a hole transport layer, or forming an electron hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer is formed, optionally a hole blocking layer is formed on the emission layer and on the electron transport layer an electro injection layer is formed,
- and finally a cathode electrode is formed,
- optional a hole injection layer, a hole transport layer, and a hole blocking layer, formed in that order between the first anode electrode and the emission layer,
- an electron injection layer is formed between the electron transport layer and the cathode electrode.

The method comprises forming an electron injection layer on the organic semiconducting layer. According to various embodiments, the OLED may have the following layer structure, wherein the layers having the following order: anode, hole injection layer, first hole transport layer, second hole transport layer, emission layer, optional hole blocking layer, an electron transport layer, the electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

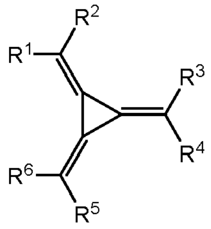 (XX) 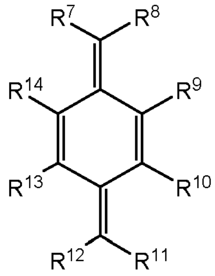 (XXIa) 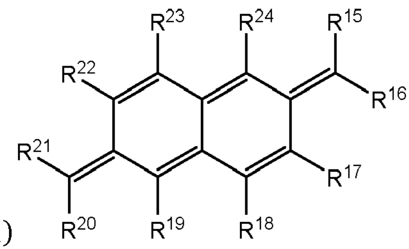 (XXIb),

wherein $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

GENERAL DEFINITIONS

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl,

preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

The organic electroluminescent device (OLED) may be a bottom- or top-emission device. The organic electroluminescent device (OLED) may emit the light trough a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of', "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.
Preferably, the organic semiconducting layer comprising the compound of Formula (I) is essentially non-emissive or non-emitting.
The operating voltage, also named U, is measured in Volt (V) at 10 milliAmpere per square centimeter ($mA/cm^2$).
The candela per Ampere efficiency, also named cd/A efficiency is measured in candela per ampere at 10 milliAmpere per square centimeter ($mA/cm^2$).
The external quantum efficiency, also named EQE, is measured in percent (%).
The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.
The highest occupied molecular orbital, also named HOMO, and lowest unoccupied molecular orbital, also named LUMO, are measured in electron volt (eV).
The term "OLED", "organic light emitting diode", "organic light emitting device", "organic optoelectronic device" and "organic light-emitting diode" are simultaneously used and have the same meaning.
The term "life-span" and "lifetime" are simultaneously used and have the same meaning.
The anode and cathode may be described as anode electrode / cathode electrode or anode electrode / cathode electrode or anode electrode layer / cathode electrode layer.
Room temperature, also named ambient temperature, is 23°C.

BRIEF DESCRIPTION OF THE DRAWINGS

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention;

FIG. 2 is a schematic sectional view of an OLED, according to an exemplary embodiment of the present invention.

FIG. 3 is a schematic sectional view of a tandem OLED comprising a charge generation layer, according to an exemplary embodiment of the present invention.

Reference will now be made in detail to the exemplary embodiments of the present invention, examples of which are illustrated in the accompanying drawings, wherein like reference numerals refer to the like elements throughout. The exemplary embodiments are described below, in order to explain the aspects of the present invention, by referring to the figures.
Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.
FIG. 1 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.
Instead of a single electron transport layer 160, optionally an electron transport layer stack (ETL) can be used.
Fig. 2 is a schematic sectional view of an OLED 100, according to another exemplary embodiment of the present invention. Fig. 2 differs from Fig. 1 in that the OLED 100 of Fig. 2 comprises an electron blocking layer (EBL) 145 and a hole blocking layer (HBL) 155.
Referring to Fig. 2, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.
Fig. 3 is a schematic sectional view of a tandem OLED 200, according to another exemplary embodiment of the present invention. Fig. 3 differs from Fig. 2 in that the OLED 200 of Fig. 3 further comprises a charge generation

layer (CGL) and a second emission layer (151).

Referring to Fig. 3, the OLED 200 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

While not shown in Figs. 1 to 3, a sealing layer may further be formed on the cathode electrodes 190, in order to seal the OLEDs 100 and 200. In addition, various other modifications may be applied thereto.

Hereinafter, the embodiments are illustrated in more detail with reference to examples. However, the present disclosure is not limited to the following examples. Reference will now be made in detail to the exemplary aspects.

EXPERIMENTAL PART

All compounds prepared below are thermally stable and final purification thereof was done by preparative vacuum sublimation.

Preparation of exemplary compounds

**2,4-diphenyl-6-(1-(3',4',5'-triphenyl-[1,1':2',1"-terphenyl]-3-yl)naphthalen-2-yl)-1,3,5-triazine (E1)**

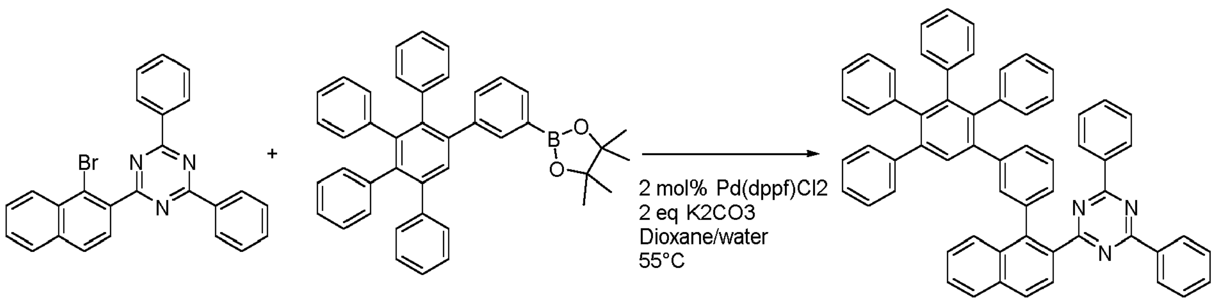


3-neck round-bottom flask was flushed with nitrogen and charged with 2-(1-bromonaphthalen-2-yl)-4,6-diphenyl-1,3,5-triazine (CAS 1642848-97-2, 1 eq, 15.0 g), 4,4,5,5-tetramethyl-2-(3',4',5'-triphenyl-[1,1':2',1"-terphenyl]-3-yl)-1,3,2-dioxaborolane (CAS 872118-08-6, 1 eq, 20 g), [1,1'-bis(diphenylphosphino)ferrocene]dichloropalladium(II) (CAS 72287-26-4, 0.02 eq, 0,50 g), potassium carbonate (CAS 584-08-7, 2 eq, 9.44 g). Deaerated mixture of 170 ml dioxane and 34 ml water was added. Reaction was running at 55°C under a nitrogen atmosphere overnight. Next day, dense yellow suspension was formed. The reaction mixture was cooled down, the precipitate was filtered and washed with methanol and water, and dried at low pressure. The raw product was dissolved at elevated temperature in 300 ml chlorobenzene and filtered hot through a silica pad. The solvent was rotary evaporated to residual volume of the solution about 200 mL and the formed precipitate was filtered. Light yellow crystalline powder was obtained. Yield: 22.5 g (81 %).

**2,4-diphenyl-6-(1-(3-(3,5,6-triphenylpyrazin-2-yl)phenyl)naphthalen-2-yl)-1,3,5-triazine (E2)**

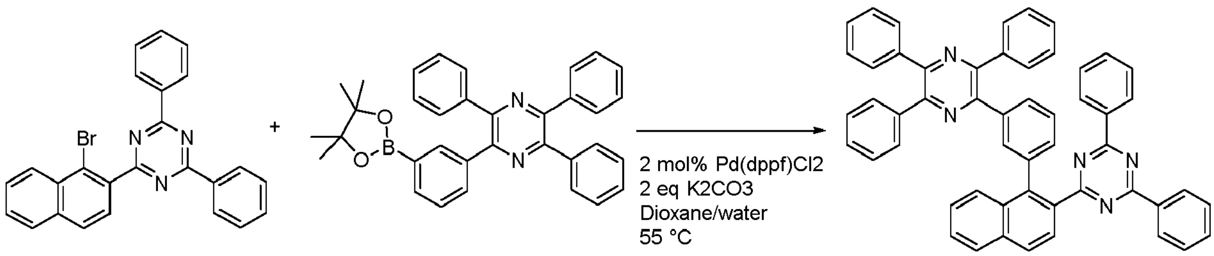

3-neck round-bottom flask was flushed with nitrogen and charged with 2-(1-bromonaphthalen-2-yl)-4,6-diphenyl-1,3,5-triazine (CAS 1642848-97-2, 1 eq, 15.0 g), 2,3,5-triphenyl-6-(3-(4,4,5,5-tetramethyl-1,3,2-dioxaborolan-2-yl)phenyl)pyrazine (CAS 2396743-64-7, 1 eq, 18.34 g), [1,1'-bis(diphenylphosphino)ferrocene]dichloropalladium(II) (CAS 72287-26-4, 0.02 eq, 0.5 g), potassium carbonate (CAS 584-08-7, 2 eq, 9.44 g). Deaerated mixture of 170 mL dioxane and 34 mL water was added. Reaction was running at 55°C under a nitrogen atmosphere overnight. Then the reaction mixture was cooled down and extracted with chloroform, the extract washed with water and filtered through silica pad. Solvent was evaporated and the product was recrystallized from chlorobenzene. Light yellow powder. Yield: 14.94 g (59 %).

**2,4-diphenyl-6-(1-(3',4',5'-triphenyl-[1,1':2',1"-terphenyl]-4-yl)naphthalen-2-yl)-1,3,5-triazine (E3)**

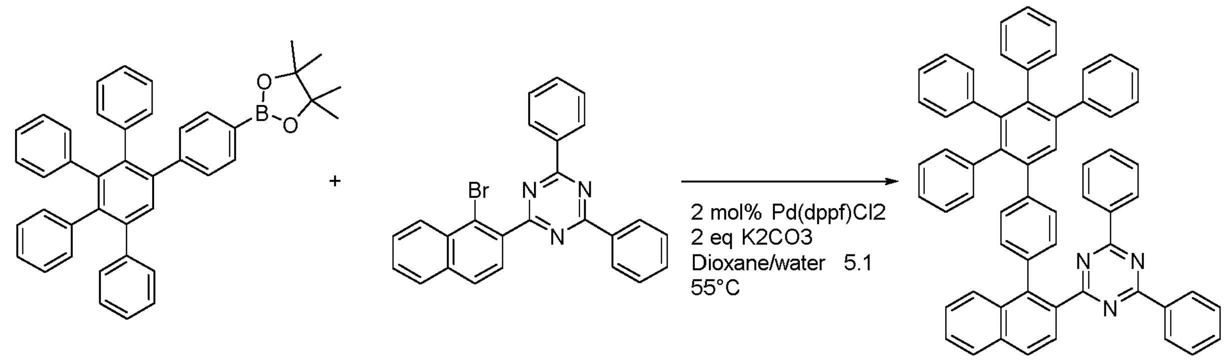


3-neck round-bottom flask was flushed with nitrogen and charged with 4,4,5,5-tetramethyl-2-(3',4',5'-triphenyl-[1,1':2',1"-terphenyl]-4-yl)-1,3,2-dioxaborolane (CAS 2122311-15-1, 1 eq, 25.0 g), 2-(1-bromonaphthalen-2-yl)-4,6-diphenyl-1,3,5-triazine (CAS 1642848-97-2, 1 eq, 18.75 g), [1,1'-bis(diphenylphosphino)ferrocene]dichloropalladium(II) (CAS 72287-26-4, 0.02 eq, 0.63 g), potassium carbonate (CAS 584-08-7, 2 eq, 11.8 g). Deaerated mixture of 214 mL dioxane and 43 mL water was added. Reaction was running at 55°C under a nitrogen atmosphere overnight. Thick yellow suspension formed, the reaction mixture was cooled down; the precipitate was filtered and washed with water and methanol. The raw product was dissolved at elevated temperature in 500 mL chlorobenzene and filtered hot through a silica pad. The solvent was evaporated and the product was recrystallized from 240 mL toluene/chlorobenzene mixture in volume ratio 6:4. White crystalline solid was obtained. Yield: 25.41 g (73 %).

**2-(1-(3',5'-diphenyl-[1,1':4',1"-terphenyl]-4-yl)naphthalen-2-yl)-4,6-diphenyl-1,3,5-triazine (E4)**

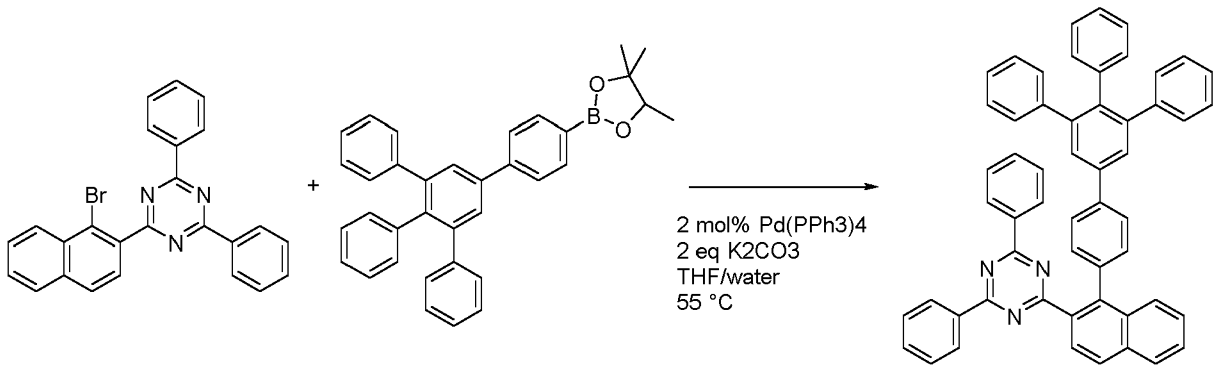


3-neck round-bottom flask was flushed with nitrogen and charged with 2-(1-bromonaphthalen-2-yl)-4,6-diphenyl-1,3,5-triazine (CAS 1642848-97-2, 1 eq, 20.0 g), 2-(3',5'-diphenyl-[1,1':4',1"-terphenyl]-4-yl)-4,4,5,5-tetramethyl-1,3,2-dioxaborolane (CAS 2358709-40-5, 1.05 eq, 24.36 g), tetrakis(triphenylphosphine)palladium(0) (CAS 14221-01-3, 0.02 eq, 1.05 g) and potassium carbonate (CAS 584-08-7, 2 eq, 12.6 g). Deaerated mixture of 280 mL THF and 46 mL water was added. The reaction was running at 55°C under a nitrogen atmosphere overnight. A thick orange suspension formed.

The reaction mixture was cooled down; precipitate was filtered and washed with water and methanol. The raw product was dissolved at elevated temperature in 1300 mL chlorobenzene and hot filtered through silica pad. The solution was evaporated, the product was recrystallized from chlorobenzene and obtained as a white powder. Yield: 11.32 g (46 %).

**2,4-diphenyl-6-(4-(3',4',5'-triphenyl-[1,1':2',1"-terphenyl]-3-yl)naphthalen-1-yl)-1,3,5-triazine (E5)**

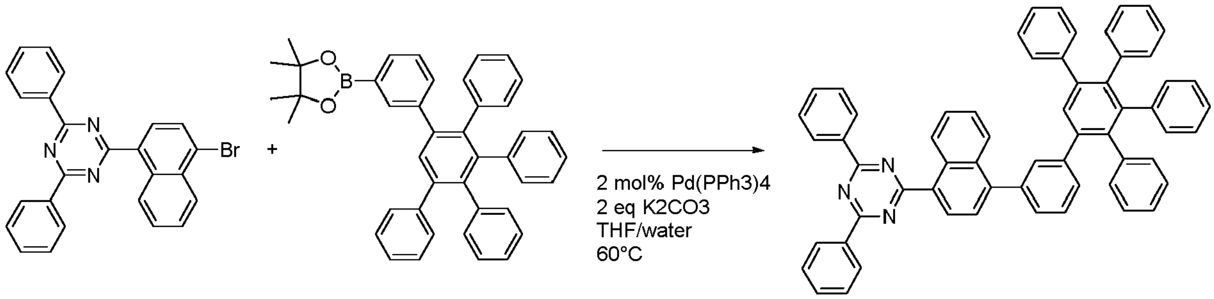


3-neck round-bottom flask was flushed with nitrogen and charged with 2-(4-bromonaphthalen-1-yl)-4,6-diphenyl-1,3,5-triazine (CAS 1800228-86-7, 1 eq, 20.0 g), 4,4,5,5-tetramethyl-2-(3',4',5'-triphenyl-[1,1':2',1"-terphenyl]-3-yl)-1,3,2-dioxaborolane (CAS 872118-08-6, 1.05 eq, 28.0 g), tetrakis(triphenylphosphine)palladium(0) (CAS 14221-01-3, 0.02 eq. 1.06 g), potassium carbonate (CAS 584-08-7, 2 eq, 12.62 g). Deaerated mixture of 340 mL THF and 47 mL water was added. Reaction was running at 60°C under a nitrogen atmosphere overnight. Then, the reaction mixture was cooled down and extracted with dichloromethane. The organic phase was filtered through silica pad, the solvent was evaporated, the crude product was recrystallized from mixture of isopropanol/cyclohexane with volume ratio 2:1 and obtained as a white powder. Yield: 18.5 g (50 %).

Supporting materials for device experiments

F1 is

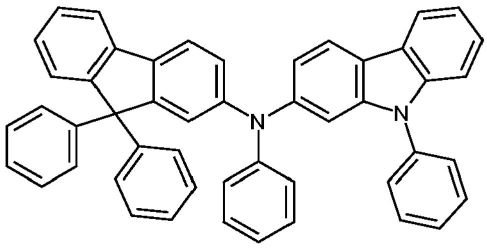

CAS 1607480-22-7

F2 is

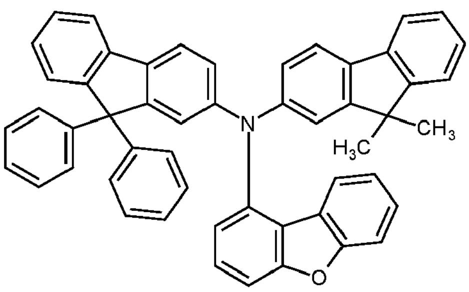

CAS 2356086-93-4

F3 is

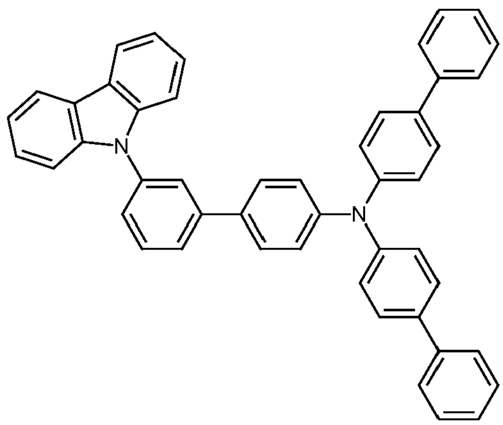

CAS 1464822-27-2

F4 is

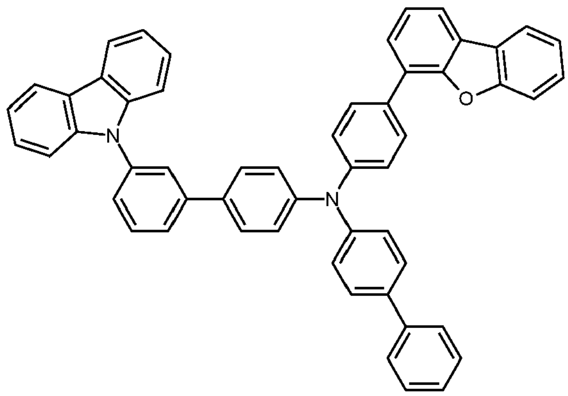

2245098-00-2

F5 is

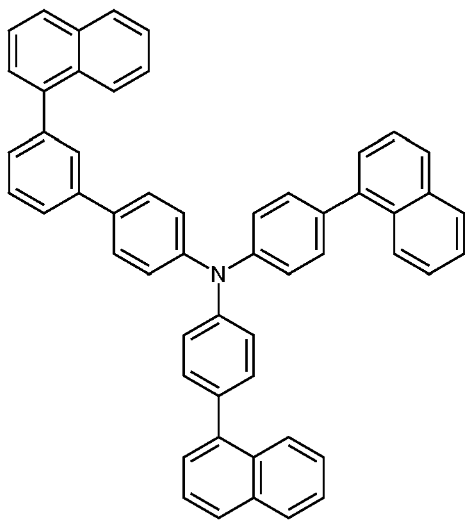

2070832-93-6

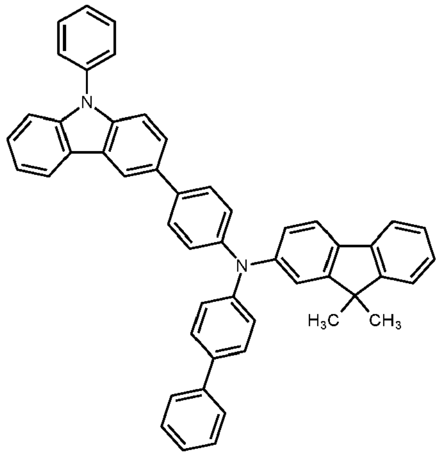

CAS 1242056-42-3

PD1 is

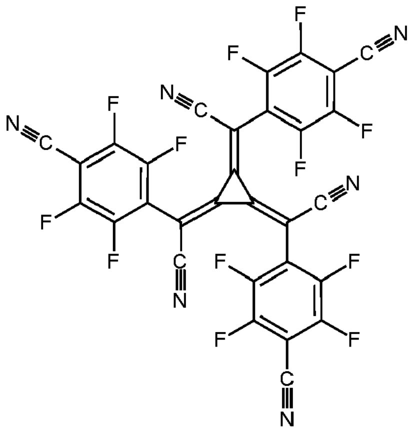

CAS 1224447-88-4

F7 is

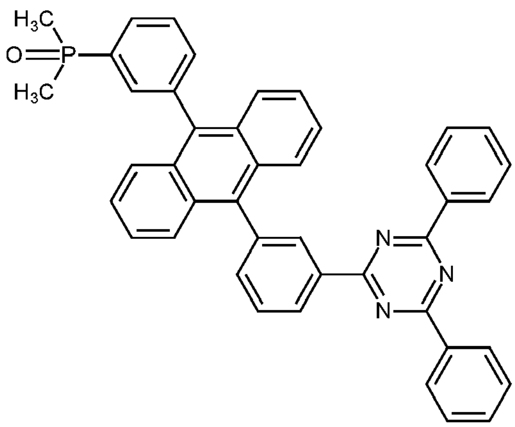

. CAS 2253724-57-9

F8 is

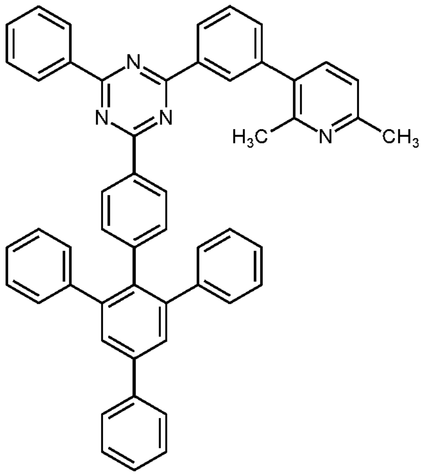

CAS 2437303-46-1

LiQ is lithium 8-hydroxyquinolinolate (CAS 850918-68-2), H09 is an emitter host and BD200 is a blue fluorescent emitter dopant, both commercially available from SFC, Korea.

General procedures

Sublimation temperature

Under nitrogen in a glovebox, 0.5 to 5 g compound are loaded into the evaporation source of a sublimation apparatus. The sublimation apparatus consist of an inner glass tube consisting of bulbs with a diameter of 3 cm which are placed inside a glass tube with a diameter of 3.5 cm. The sublimation apparatus is placed inside a tube oven (Creaphys DSU 05/2.1). The sublimation apparatus is evacuated via a membrane pump (Pfeiffer Vacuum MVP 055-3C) and a turbo pump (Pfeiffer Vacuum THM071 YP). The pressure is measured between the sublimation apparatus and the turbo pump using a pressure gauge (Pfeiffer Vacuum PKR 251). When the pressure has been reduced to $10^{-5}$ mbar, the temperature is increased in increments of 10 to 30 K till the compound starts to be deposited in the harvesting zone of the sublimation apparatus. The temperature is further increased in increments of 10 to 30 K till a sublimation rate is achieved where the compound in the source is visibly depleted over 30 min to 1 hour and a substantial amount of compound has accumulated in the harvesting zone.

The sublimation temperature, also named $T_{sub1}$, is the temperature inside the sublimation apparatus at which the compound is deposited in the harvesting zone at a visible rate and is measured in degree Celsius.

In the context of the present invention, the term "sublimation " may refer to a phase transfer from solid state to gas phase or from liquid state to gas phase.

Decomposition temperature

The decomposition temperature, also named $T_{dec}$, is determined in degree Celsius.

The decomposition temperature is measured by loading a sample of 9 to 11 mg into a Mettler Toledo 100 μL aluminum pan without lid under nitrogen in a Mettler Toledo TGA-DSC 1machine. The following heating program was used: 25°C isothermal for 3 min; 25°C to 600°C with 10 K/min.

The decomposition temperature was determined based on the onset of the decomposition in TGA.

Rate onset temperature

The rate onset temperature ($T_{RO}$) is determined by loading 100 mg compound into a VTE source. As VTE source a point source for organic materials may be used as supplied by Kurt J. Lesker Com-pany (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behaviour.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected. The HOMO and LUMO levels are recorded in electron volt (eV).

OLED evaluation

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in $cd/m^2$ using an Instrument Systems CAS-140CT array spectrometer (calibrated by Deutsche Akkreditierungsstelle (DAkkS)) for each of the voltage values. The cd/A efficiency at 10 $mA/cm^2$ is determined by interpolating the luminance-voltage and current-voltage characteristics, respectively.

Lifetime LT of the device is measured at ambient conditions (20°C) and 30 $mA/cm^2$, using a Keithley 2400 sourcemeter, and recorded in hours.

The brightness of the device is measured using a calibrated photo diode. The lifetime LT is defined as the time till the brightness of the device is reduced to 97 % of its initial value.

To determine the voltage stability over time U(100h)-(1h) and U(100h-50h), a current density of at 30 $mA/cm^2$ was applied to the device. The operating voltage was measured after 1 hour, after 50 hours and after 100 hours, followed by calculation of the voltage stability for the time period of 1 hour to 100 hours and for a time period of 50 hours to 100 hours.

Voltage stability

OLEDs are driven by constant current circuits. Those circuits can supply a constant current over a given voltage range. The wider the voltage range, the wider the power losses of such devices. Hence, the change of driving voltage upon driving needs to be minimized.

The driving voltage of an OLED is temperature dependent. Therefore, voltage stability needs to be judged in thermal equilibrium. Thermal equilibrium is reached after one hour of driving.

Voltage stability is measured by taking the difference of the driving voltage after 50 hours and after 1 hour driving at a constant current density. Here, a current density of 30 $mA/cm^2$ is used. Measurements are done at room temperature.

$$dU\ [V] = U(50\ h,\ 30\ mA/cm^2) - U(1\ h,\ 30\ mA/cm^2)$$

Examples

1) Blue fluorescent top emission OLED

Table 1a schematically describes the model device. The device was prepared by vacuum thermal evaporation (VTE) process.

Table 1a

| Layer | Material | d [nm] |
|---|---|---|
| Anode | Ag | 100 |
| HIL | HTL matrix:PD1 (92:8 v/v) | 10 |
| HTL | HTL matrix | 128 |
| EBL | EBL matrix | 5 |
| EML | H09:BD200 (99:1 v/v) | 20 |
| HBL | C1 or E1 | 5 |
| ETL | F7:F8:LiQ (10:40:50 v/v) | 31 |
| EIL | Yb | 2 |
| Cathode | Ag:Mg (90:10) | 13 |

The results are given in Table 1b

Table 1b

| HTL matrix | EBL matrix | aETL | CIEy | j [mA/cm$^2$] | Voltage [V] | | CEff/CIEy [cd/A] | | LT97 at 1110 cd/m$^2$ [h] | |
|---|---|---|---|---|---|---|---|---|---|---|
| F6 | F3 | *C1*<br>**E1** | 0,054<br>0,053 | 10,23<br>10,28 | 3.30<br>3,31 | 100%<br>100% | 199<br>202 | 100%<br>101% | 358<br>399 | 100%<br>**111%** |
| F1 | F4 | *C1*<br>**E1** | 0,054<br>0,054 | 10,18<br>10,04 | 3,31<br>3,31 | 100%<br>100% | 198<br>203 | 100%<br>102% | 213<br>260 | 100%<br>**122%** |
| F2 | F3 | *C1*<br>**E1** | 0,047<br>0,046 | 10,91<br>10,95 | 3,20<br>3,21 | 100%<br>100% | 216<br>217 | 100%<br>101% | 262<br>298 | 100%<br>**114%** |
| F1 | F5 | *C1*<br>**E1** | 0,052<br>0,052 | 11,77<br>11,43 | 3,26<br>3,26 | 100%<br>100% | 181<br>186 | 100%<br>103% | 159<br>205 | 100%<br>**129%** |

With various materials on the p-side, the representative compound E1 provided significant lifetime improvement without any negative impact on operational voltage and/or efficiency.

The features disclosed in the foregoing description and in the dependent claims may, both separately and in any combination thereof, be material for realizing the aspects of the disclosure made in the independent claims, in diverse forms thereof.

## Claims

**1.** Compound having the formula (I)

$$E\text{-}A^1\text{-}A^2\text{-}A^3 \qquad (I),$$

wherein in formula (I)

- E is selected from the group consisting of a substituted or unsubstituted $C_4$-$C_{70}$ heteroaryl comprising a diazine group, a substituted or unsubstituted $C_3$-$C_{70}$ heteroaryl comprising a triazine group, a $C_{10}$-$C_{70}$ aryl comprising at least two condensed benzene rings, and a $C_4$-$C_{70}$ heteroaryl comprising at least two condensed aromatic rings;

  - wherein in case that E is substituted or unsubstituted $C_4$-$C_{70}$ heteroaryl comprising a diazine group E is bonded to $A^1$ via a direct bond between $A^1$ and the diazine group;
  - wherein in case that E is substituted or unsubstituted $C_4$-$C_{70}$ heteroaryl comprising a triazine group E is bonded to $A^1$ via a direct bond between $A^1$ and the triazine group;
  - wherein in case that E is $C_{10}$-$C_{70}$ aryl comprising at least two condensed benzene rings E is bonded to $A^1$ via a direct bond between $A^1$ and one of the condensed benzene rings; and
  - wherein in case that E is $C_4$-$C_{70}$ heteroaryl comprising at least two condensed aromatic rings E is bonded to $A^1$ via a direct bond between $A^1$ and one of the condensed aromatic rings;

- $A^1$ is selected from the group consisting of a substituted or unsubstituted $C_{10}$-$C_{70}$ arylene comprising 2 or 3 condensed rings and a substituted or unsubstituted $C_2$-$C_{70}$ a heteroarylene comprising 2 or 3 condensed rings; wherein $A^1$ is bonded to E via a direct bond between one of the condensed rings and E; and wherein $A^1$ is bonded to $A^2$ via a direct bond between one of the condensed rings and $A^2$;
- $A^2$ is selected from the group consisting of a substituted or unsubstituted carbocyclic aromatic ring, a substituted or unsubstituted heterocyclic aromatic ring, a substituted or unsubstituted condensed bicyclic aromatic system and a substituted or unsubstituted condesned bicyclic heteroaromatic system; wherein $A^2$ is bonded to $A^3$ via a direct bond between one of the aromatic or heteroaromatic rings and $A^3$; and
- $A^3$ is selected from the group consisting of a monocyclic aryl and a monocyclic heteroaryl, wherein $A^3$ is substituted with at least three groups independently selected from the group consisting of substituted or unsubstituted $C_6$-$C_{18}$ aryl and $C_2$-$C_{18}$ heteroaryl.

**2.** Compound according to claim 1, wherein E is selected from the group consisting of pyrimidine, pyrazine, pyridazine, triazine, naphthalene and aza-analogs thereof, especially quinazoline, anthracene and aza-analogs thereof, phenanthrene and aza-analogs thereof, specially benzoquinazoline, fluorene and aza-analogs thereof, benzofluorene and aza-analogs thereof, dibenzofluorene and aza-analogs thereof, naphtofluorene and aza-analogs thereof, benzonaphtofluorene and aza-analogs thereof, dinaphtofluorene and aza-analogs thereof, benzimidazole and aza analogs thereof, benzoxazole and aza analogs thereof, benzothiazole and aza analogs thereof, pyrido-imidazole and aza analogs thereof, benzofurane and aza-analogs thereof, benzothiophene and aza-analogs thereof, dibenzofurane and aza-analogs thereof, dibenzothiophene and aza-analogs thereof, naphtofurane and aza-analogs thereof, naphtothiophene and aza-analogs thereof, naphtobenzofurane and aza-analogs thereof, naphtobenzothiophene and aza-analogs thereof, dinaphtofurane and aza-analogs thereof, dinaphtothiophene and aza-analogs thereof, aryl consisting of 4, 5, 6 or 7 condensed rings and aza-analogs thereof, and heteroaryl consisting of 3, 4, 5, 6 or 7 condensed rings and comprising 1, 2 or 3 ring atoms independently selected from O and S, and aza-analogs of the heteroaryl consisting of 3, 4, 5, 6 or 7 condensed rings and comprising 1, 2 or 3 ring atoms independently selected from O and S, wherein the groups may be substituted or unsubstituted, respectively, especially with one or more phenyl and/or pyridyl groups, wherein the phenyl and/or pyridyl groups may be substituted or unsubstituted.

**3.** Compound according to claim 1 or 2, wherein E is selected from the group consisting of pyrimidine, pyrazine, cinnoline, quinoline, phtalazine, quinoxaline, quinazoline, benzoquinazoline, pyrido-imidazole, benzimidazole, pyrido-benzimidazole and 1,3,5-triazine, wherein the groups may be substituted or unsubstituted, respectively, especially may be substituted with one or more unsubstituted phenyl.

**4.** Compound according to any of the preceding claims, wherein $A^1$ is selected from the group consisting of naphtalene-diyl and aza-analogs thereof, anthracene-diyl and aza-analogs thereof, phenanthrene-diyl and aza-analogs thereof, fluorene-diyl and aza-analogs thereof, benzothiophene-diyl and aza-analogs thereof, benzofurane-diyl and aza-analogs thereof, naphtofurane-diyl and aza-analogs thereof, naphtothiophene-diyl and aza-analogs thereof, dibenzofurane-diyl aza-analogs thereof, and dibenzothiophene-diyl and aza-analogs thereof.

**5.** Compound according to any of the preceding claims, wherein $A^1$ is selected from the group consisting of naphtalene-1,2-diyl, naphtalene-1,3-diyl, naphtalene-1,4-diyl, naphtalene-1,5-diyl, naphtalene-1,6-diyl, naphtalene-1,7-diyl, naphtalene-1,8-diyl, naphtalene-2,3-diyl, and naphtalene-2,6-diyl, naphtalene-2,7-diyl, and phenanthrene-9,10-diyl.

**6.** Compound according to any of the preceding claims, wherein $A^2$ is selected from the group consisting of phenylene, thiophenylene, furanylene, pyrazolylene, imidazolylene, triazolylene, pyridinylene, pyrimidinylene, pyridazinylene,

pyrazinylene and triazinylene.

7. Compound according to any of the preceding claims, wherein $A^2$ is selected from the group consisting of o-phenylene, m-phenylene, p-phenylene, and pyridine-3,5-ylene.

8. Compound according to any of the preceding claims, wherein $A^3$ is selected from the group consisting of phenyl, thiophenyl, furanyl, pyridinyl, pyrimidinyl, pyridazinyl, and pyrazinyl.

9. Compound according to any of the preceding claims, wherein $A^3$ is selected from the group consisting of phenylene, pyridinyl, pyrimidinyl and pyrazinyl.

10. Compound according to any of the preceding claims, wherein the at least three groups by which $A^3$ is substituted are independently selected from the group consisting of unsubstituted phenyl, phenyl substituted with one or more hydrocarbyl groups, wherein the total number of carbon atoms in the phenyl substituted with one or more hydrocarbyl groups is from 7 to 18, pyridyl, pyridyl substituted with one or more hydrocarbyl groups, wherein the total number of carbon atoms in the phenyl substituted with one or more hydrocarbyl groups is from 7 to 18, and CN-substituted phenyl, especially phenyl substituted with one CN.

11. Compound according to any of the preceding claims, wherein the compound of formula (I) is selected from the following compounds E1 to E20

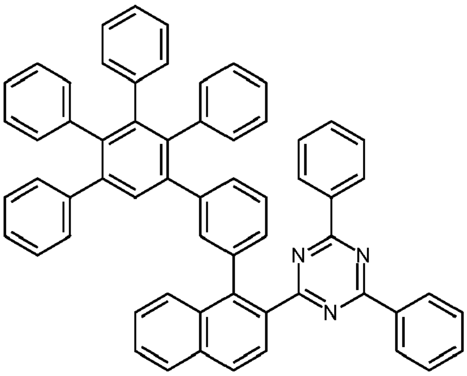
E1

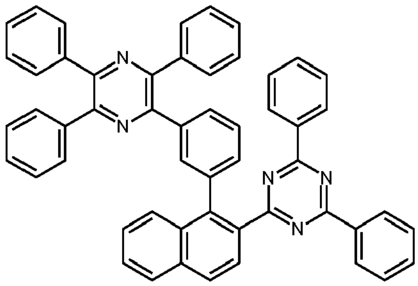
E2

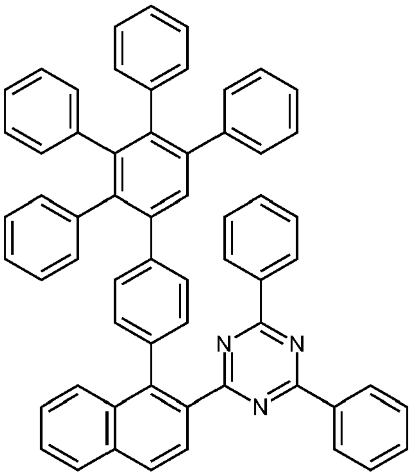
E3

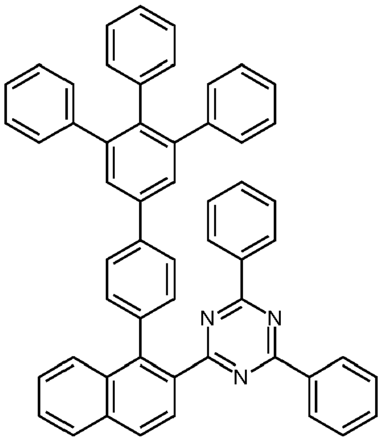
E4

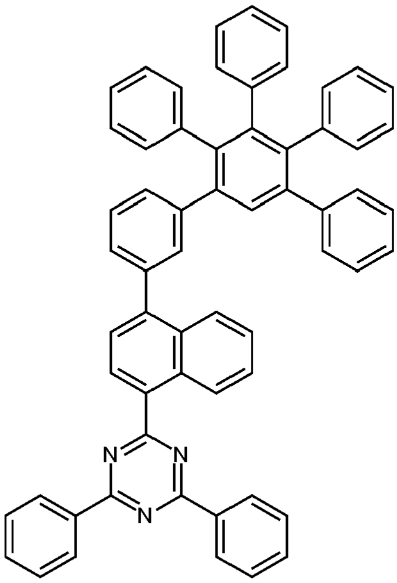

E5

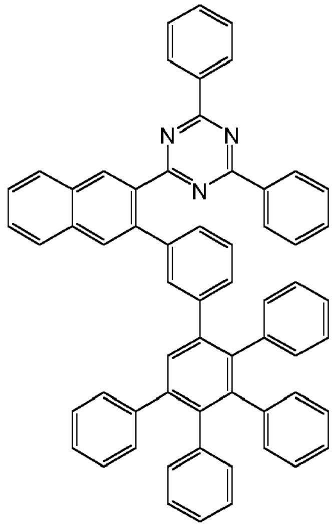

E6

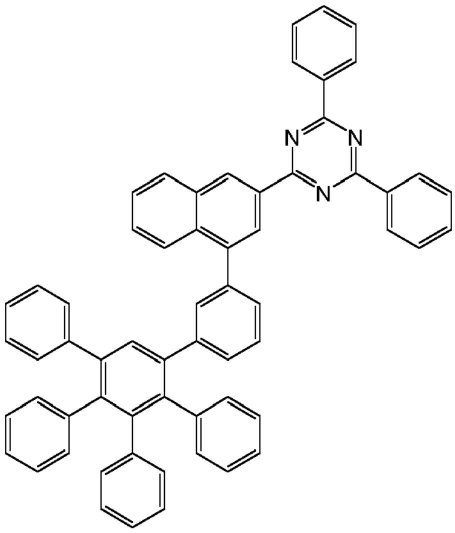

E7

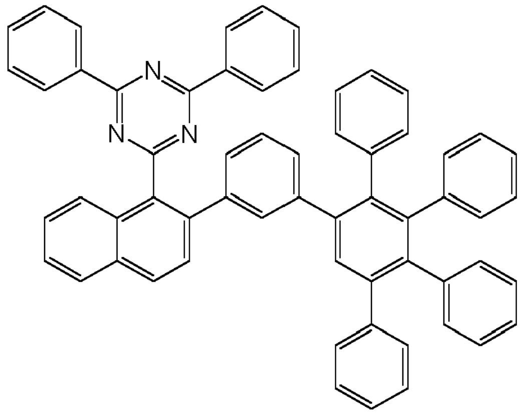

E8

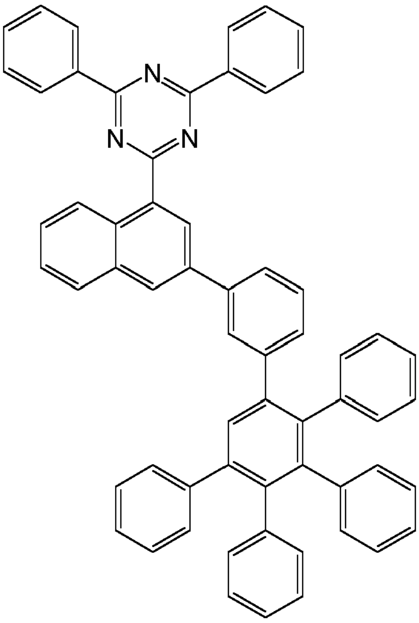

E9

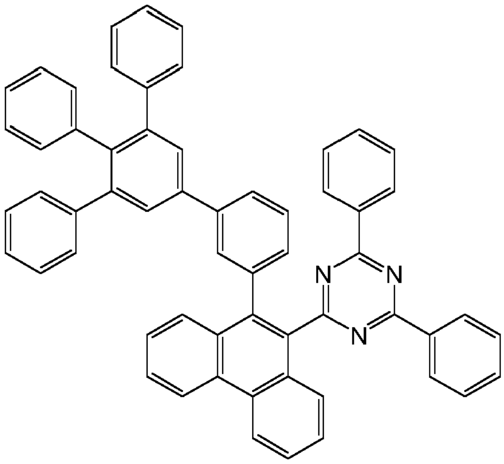

E10

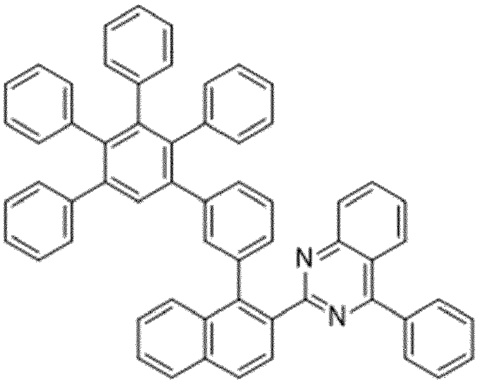

E11

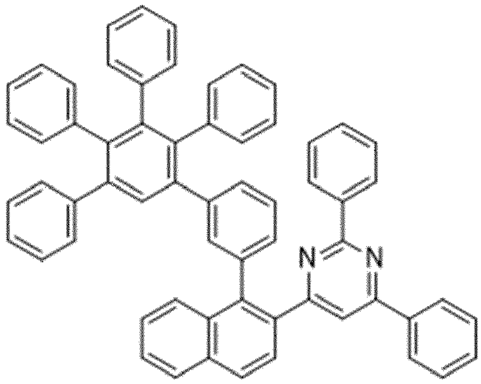

E12

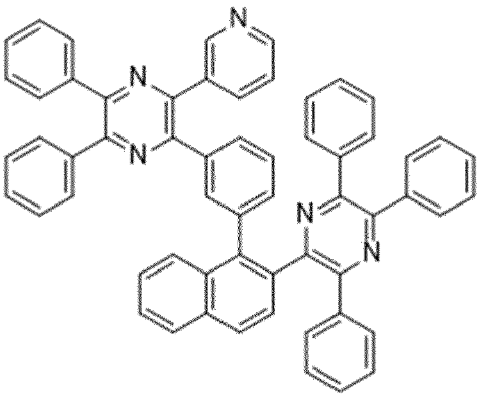

E13

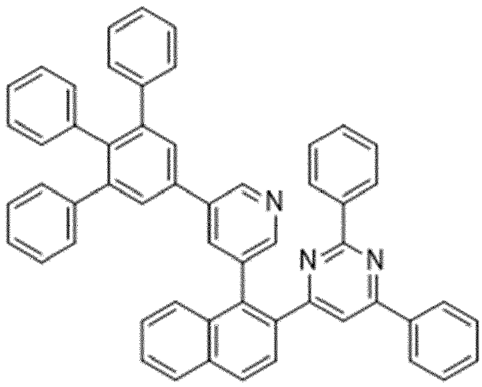

E14

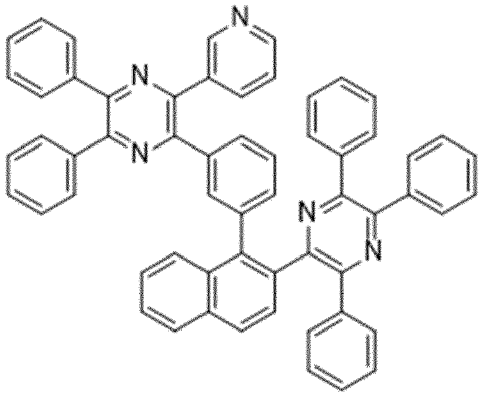
E15

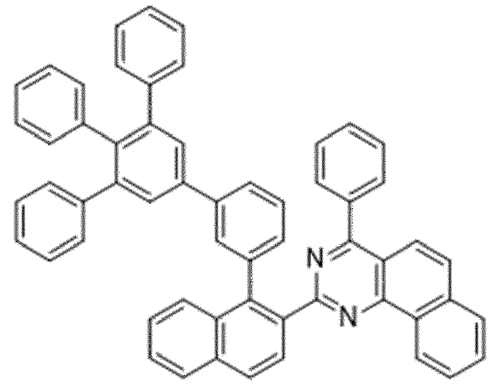
E16

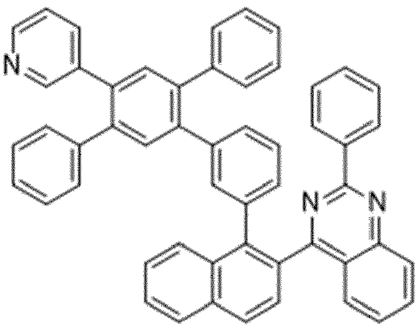
E17

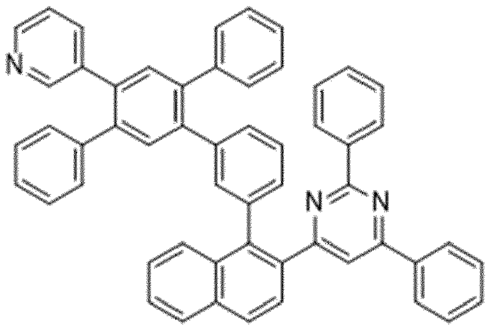
E18

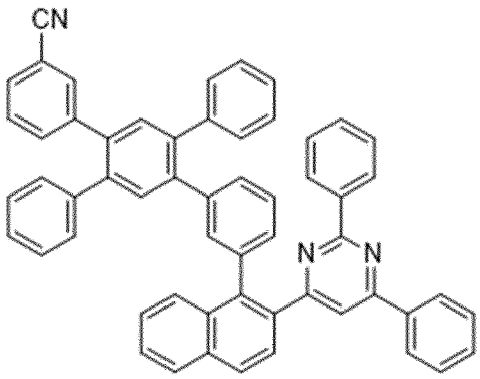
E19

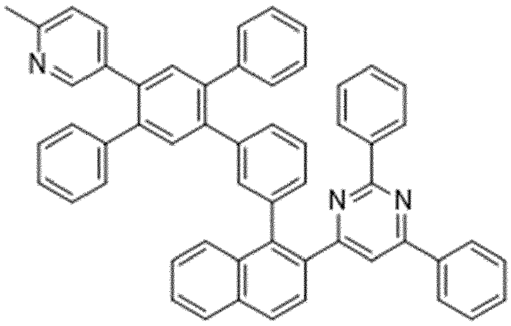
E20.

**12.** Semiconducting material comprising the compound of formula (I) according to any of the preceding claims.

**13.** Electronic device comprising at least one layer comprising the compound of formula (I) according to any of the claims 1 to 11.

**14.** Electronic device according to claim 13, wherein

- the electronic device comprises a first electrode, a second electrode, the layer comprising the compound of formula (I), and a light emitting layer;
- the compound of formula (I) and the light emitting layer are arranged between the first electrode and the second electrode;
- the layer comprising the compound of formula (I) is a semiconducting layer;
- the layer comprising the compound of formula (I) is arranged between the first electrode and the second electrode; and
- the layer comprising the compound of formula (I) is in direct contact with the light emitting layer.

**15.** Display device comprising a light emitting diode, wherein the light emitting diode comprises the compound of formula (I) according to any of the claims 1 to 11.

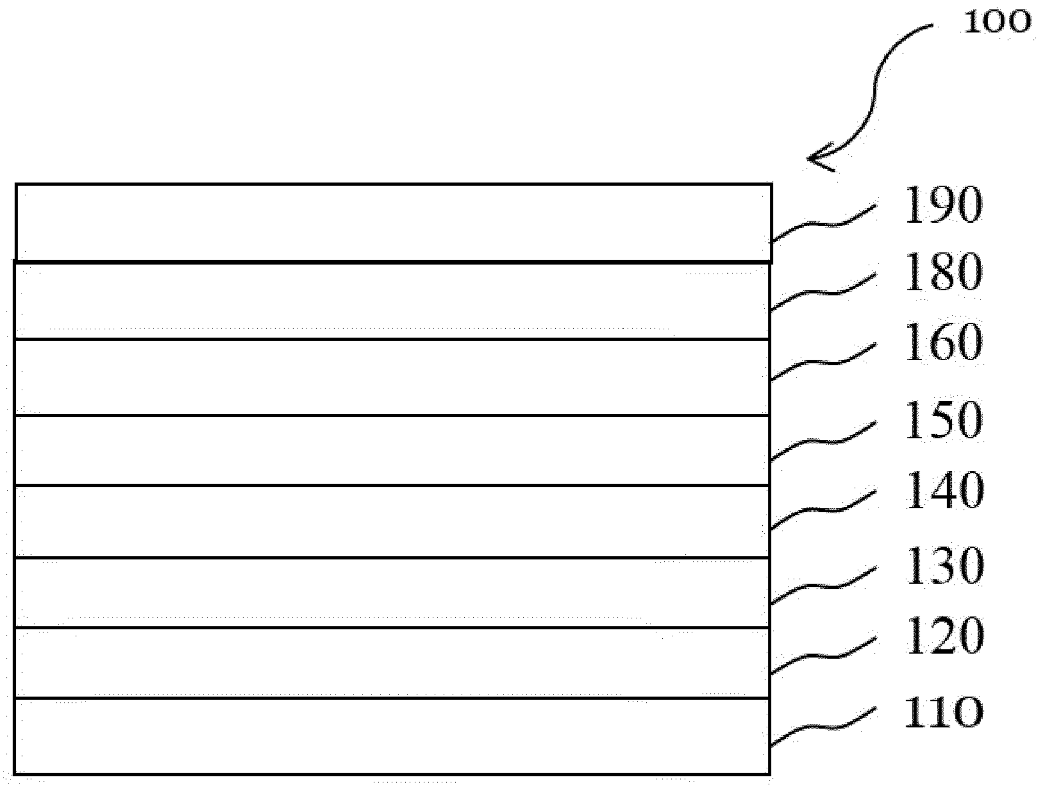


Fig.1

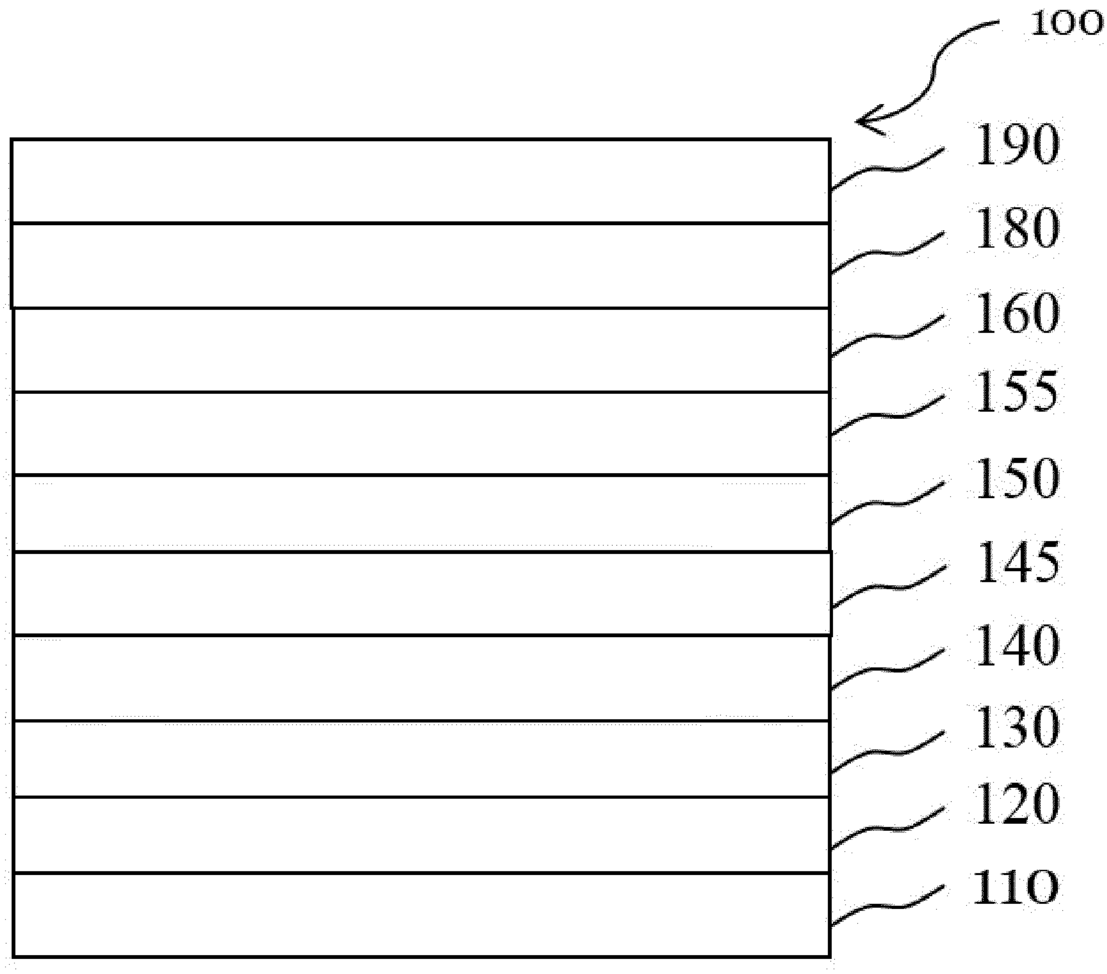


Fig.2

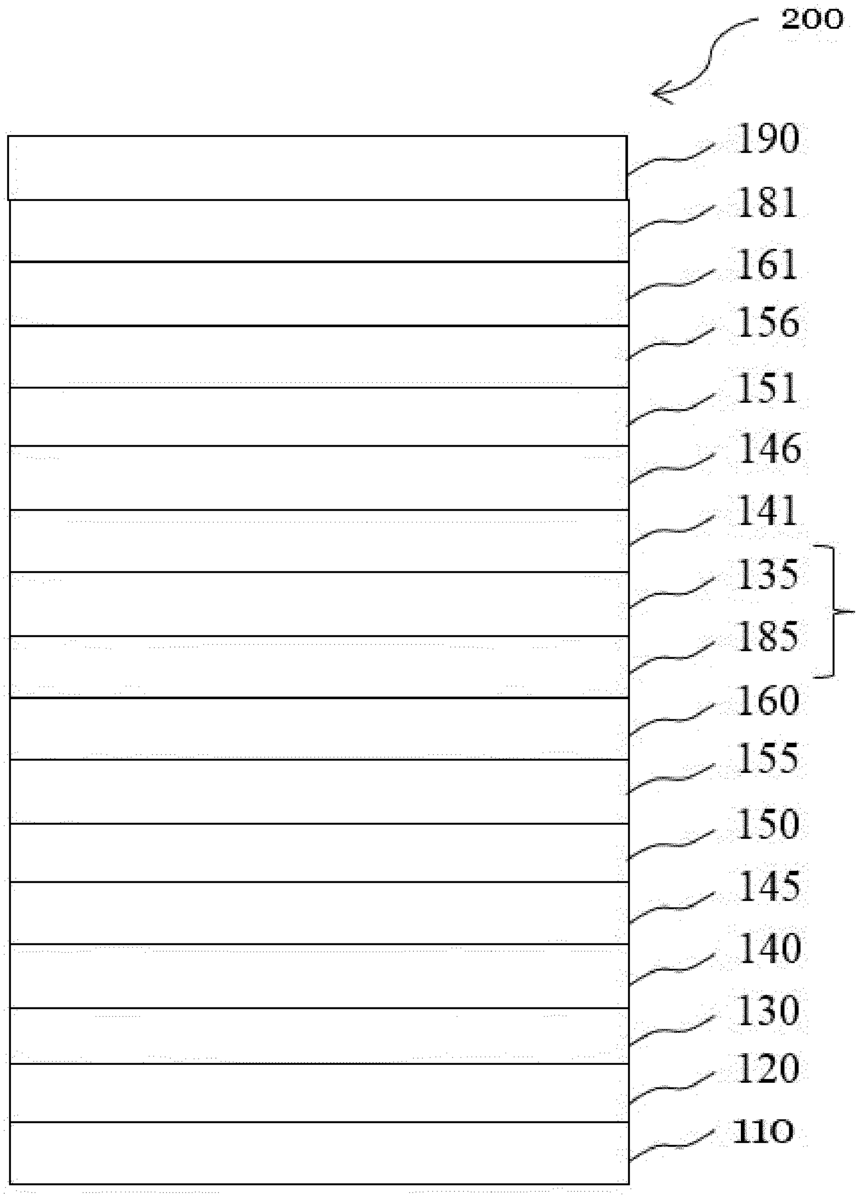
200
190
181
161
156
151
146
141
135
185
160
155
150
145
140
130
120
110

Fig.3

Europäisches Patentamt
European Patent Office
Office européen des brevets

# EUROPEAN SEARCH REPORT

Application Number
EP 21 21 4412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/154713 A1 (NOVALED GMBH [DE]) 15 August 2019 (2019-08-15) * compounds A37, A38, A45, A46, A61, A62, A69, A70, A76, A77, A84, A85, A91, A92, A106, A109, A115 and A116 on pages 18ff.; claims 1, 5, 8-15 * | 1-15 | INV. C07D239/26 C07D239/74 C07D241/12 C07D251/24 H01L51/00 |
| X | WO 2020/260406 A1 (NOVALED GMBH [DE]) 30 December 2020 (2020-12-30) * claims; tables 1A (pages 57-58); compounds (1a), D35 * | 1-9, 12-15 | |
| X | WO 2020/020602 A1 (CYNORA GMBH [DE]) 30 January 2020 (2020-01-30) * two compounds listed as 1st and 2nd compound of the 3rd row on page 80; claims * | 1-3, 6-10, 12-15 | |
| X | US 2019/198774 A1 (JUN MIEUN [KR] ET AL) 27 June 2019 (2019-06-27) * claims; example 4 (paragraphs 404ff.); compounds 24, 25 (page 19) * | 1-3, 5-10, 12-15 | TECHNICAL FIELDS SEARCHED (IPC) C07D |
| X | KR 2009 0079134 A (LG CHEMICAL LTD [KR]) 21 July 2009 (2009-07-21) * various compounds of claim 10, e.g. those compounds 1-8, 1-9, 1-15, 1-35, 1-51, 1-99, 2-8, 2-9, 2-15, 2-36, 2-43, 2-51 and 2-99 prepared as shown on pages 85ff, see paragraphs 195-222 and 252-284; claims * | 1,2,4-7, 10,12-15 | |

-/--

The present search report has been drawn up for all claims

1

| Place of search | Date of completion of the search | Examiner |
|---|---|---|
| Munich | 6 May 2022 | Stroeter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 21 4412

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/303655 A1 (HUH JUNGOH [KR] ET AL) 24 September 2020 (2020-09-24)<br>* 1st compound on page 8; 2nd compound on the right-hand column of page 11; claims *<br>----- | 1,2, 4-10, 12-15 | |
| X | CN 112 142 672 A (JILIN OPTICAL & ELECTRONIC MATERIALS CO LTD) 29 December 2020 (2020-12-29)<br>* paragraphs [0023], [0025], [0092]; claims; compounds 59, 82, 83 *<br>----- | 1-4,6,7, 10,12-15 | |

TECHNICAL FIELDS SEARCHED (IPC)

The present search report has been drawn up for all claims

1

| Place of search | Date of completion of the search | Examiner |
|---|---|---|
| Munich | 6 May 2022 | Stroeter, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 21 21 4412

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2022

| Patent document cited in search report | | Publication date | Patent family member(s) | | Publication date |
|---|---|---|---|---|---|
| WO 2019154713 | A1 | 15-08-2019 | CN 111712485 | A | 25-09-2020 |
| | | | EP 3524593 | A1 | 14-08-2019 |
| | | | KR 20200118097 | A | 14-10-2020 |
| | | | US 2021036230 | A1 | 04-02-2021 |
| | | | WO 2019154713 | A1 | 15-08-2019 |
| WO 2020260406 | A1 | 30-12-2020 | CN 114401958 | A | 26-04-2022 |
| | | | EP 3757100 | A1 | 30-12-2020 |
| | | | EP 3990450 | A1 | 04-05-2022 |
| | | | KR 20220024225 | A | 03-03-2022 |
| | | | WO 2020260406 | A1 | 30-12-2020 |
| WO 2020020602 | A1 | 30-01-2020 | EP 3823965 | A1 | 26-05-2021 |
| | | | WO 2020020602 | A1 | 30-01-2020 |
| US 2019198774 | A1 | 27-06-2019 | CN 109956925 | A | 02-07-2019 |
| | | | KR 20190077154 | A | 03-07-2019 |
| | | | US 2019198774 | A1 | 27-06-2019 |
| KR 20090079134 | A | 21-07-2009 | NONE | | |
| US 2020303655 | A1 | 24-09-2020 | CN 111263753 | A | 09-06-2020 |
| | | | KR 20190111824 | A | 02-10-2019 |
| | | | US 2020303655 | A1 | 24-09-2020 |
| | | | WO 2019182402 | A1 | 26-09-2019 |
| CN 112142672 | A | 29-12-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019238858 A1
- EP 1837926 A1
- WO 07107306 A1
- WO 07107356 A1
- EP 2722908 A1
- EP 1970371 A1
- WO 2013079217 A1

### Non-patent literature cited in the description

- **YASUHIKO SHIROTA ; HIROSHI KAGEYAMA.** *Chem. Rev.,* 2007, vol. 107, 953-1010
- *CHEMICAL ABSTRACTS,* 1642848-97-2
- *CHEMICAL ABSTRACTS,* 872118-08-6
- *CHEMICAL ABSTRACTS,* 72287-26-4
- *CHEMICAL ABSTRACTS,* 584-08-7
- *CHEMICAL ABSTRACTS,* 2396743-64-7
- *CHEMICAL ABSTRACTS,* 2122311-15-1
- *CHEMICAL ABSTRACTS,* 2358709-40-5
- *CHEMICAL ABSTRACTS,* 14221-01-3
- *CHEMICAL ABSTRACTS,* 1800228-86-7
- *CHEMICAL ABSTRACTS,* 850918-68-2